# EUROPEAN PATENT APPLICATION

(11) **EP 4 442 817 A1**
(43) Date of publication of application: **09.10.2024**
(21) Application number: 23166547.2
(22) Date of filing: 04.04.2023
(51) Int. Cl.: C12N 9/02, C12N 9/88, C07K 14/415, A01H 6/82, C12N 9/00, C12N 15/82, C12N 9/10, C07D 305/00, C12P 15/00, C12P 17/02

(54) **PACLITAXEL (TAXOL) BIOSYNTHESIS PATHWAY**

(71) Applicant: Max-Planck-Gesellschaft zur Förderung der Wissenschaften e.V., 80539 München (DE)
(72) Inventor: The designation of the inventor has not yet been filed
(74) Representative: Vossius & Partner Patentanwälte Rechtsanwälte mbB

(57) **Abstract**

The present invention relates to the Paclitaxel (taxol) biosynthesis pathway and in particular to methods for producing 4β,20-taxadiene, 10-deacetylbaccatin III, Baccatin III or β-phenylalanoyl-coA as well as corresponding uses. The present invention further relates to a vector, recombinant organism, a tissue, a cell, or an organelle comprising the enzymes being required for the production of 4β,20-taxadiene, 10-deacetylbaccatin III, Baccatin III or β-phenylalanoyl-coA.

## Description

The present invention relates to the Paclitaxel (taxol) biosynthesis pathway and in particular to methods for producing 4β,20-taxadiene, 10-deacetylbaccatin III, Baccatin III or β-phenylalanoyl-coA as well as corresponding uses. The present invention further relates to a vector, recombinant organism, a tissue, a cell, or an organelle comprising the enzymes being required for the production of 4β,20-taxadiene, 10-deacetylbaccatin III, Baccatin III or β-phenylalanoyl-coA.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

As the major source of bioactive small molecules, medicinal plants represent important resources for the discovery of novel principles to develop treatments against several human diseases. Since the mid-1800s, the toxicity of Taxaceae extracts to animals attracted the attention of chemists. An unusual cyclic diterpenoid (paclitaxel) was firstly isolated and characterized from the bark of the Pacific yew (*Taxus brevifolia*), and was later shown to bind primarily to β-tubulin, stabilizing the assembly of microtubules. This activity of paclitaxel results in the slowing or blocking of mitosis at the metaphase-anaphase transition, and ultimately causes the induction of apoptotic cell death. Since 1992, paclitaxel, under the brand name Taxol, was approved by the Food and Drug Administration (FDA) for medical use against several solid cancers (ovarian, breast, lung, cervical, pancreatic, and Kaposi's sarcoma)⁴. Since then, taxol, and some of its related taxane analogs (e.g., docetaxel), have become leading anti-cancer drugs with sales reported to exceed five billion per year (Onrubia et al., 2013, Curr Med Chem; 20(7):880-91.). Originally, paclitaxel was directly extracted from the twigs, bark, or needles of pacific yew, but the low yield and the amount generally required for a therapeutic cycle (around 2-3g paclitaxel/patient) have intensified the search for alternative approaches to produce paclitaxel. Total chemical synthesis of paclitaxel, achieved for the first time in 1994, was never considered as an economically sustainable alternative, given the low efficiency and the complexity of the process.

Today, paclitaxel production for medical use is based on a semisynthetic approach, starting from 10-deacetylbaccatin III isolated from natural sources (*Taxus* needles), although several alternative strategies - using plant cell cultures - have been developed. The biosynthetic steps up to Taxadiene have been successfully transferred to *E.coli,* yeast, and Tobacco chloroplasts, but the step immediately downstream, catalyzed by taxane-5α-hydroxylase (TSaOH), always represented a bottleneck of the pathway, given that more than 90% of the substrate (taxadiene) is converted to a 5(12)-oxa-3 (11)-cyclotaxane (OCT), an intermediate which can no longer re-enter the route to paclitaxel. Cell cultures from several *Taxus* species are also employed for paclitaxel production. The supply of Paclitaxel for medical use cannot be reached today entirely from natural sources.

Based on the current knowledge of the biosynthetic pathway for the production of paclitaxel the biosynthesis of Taxadiene (Taxa-4(5),11(12)-diene), a C₂₀ compound, is considered the committed step in the production of paclitaxel. Taxadiene is formed in the chloroplast by the action of taxadiene synthase, an enzyme using as substrate the geranylgeranyl pyrophosphate (GGPP) produced through the methylerythritol phosphate pathway (MEP). Taxadiene is then presumably transported to the endoplasmic reticulum (ER), where it is further modified by at least 19 enzymes. In particular, Taxadiene is hydroxylated by seven P450 hydroxylases in C1, C2, C5, C7, C9, C10, and C13 with further oxidation occurring in C9. After further modifications, including the C4β-C20 epoxidation, the formation of the oxetane ring, and the transfer of acyl groups on C5 and C4, the resulting intermediate (Baccatin III) is the target of the final decoration steps, which include the attachment of a β-phenylalanoyl moiety on C-13, additional oxidation on C-2α and the transfer of a benzoyl moiety on the same β-phenylalanoyl side chain. Twelve of these steps have been well characterized^{10,11} with one new identified T2'αOH, but for six additional hypothetical reactions, presumably catalyzed by phenylalanine-CoA ligase (PCL), taxane 1β-hydroxylase (T1βOH), taxane 9α-hydroxylase (T9αOH), taxane 9α-dioxygenase (T9oxidase), C4β-C20epoxidase and oxomutase, molecular evidence remains unclear.

Most research efforts in the past few years focused on engineering a more efficient version of TSaOH, partially restoring its catalytic specificity towards taxadiene-5α-ol. Despite the recent release of the genomic sequence of *Taxus,* the transfer of the full paclitaxel biosynthetic pathway to microbial hosts has not been achieved yet. The missing knowledge of the genes involved in several specific metabolic steps of paclitaxel biosynthesis has rendered it difficult to engineer the full pathway.

However, the entire biosynthetic pathway for the production of paclitaxel is still not yet fully elucidated, so that the optimization of its synthesis in *Taxus* cell cultures remains an obstacle. It is concluded in the review article Tong, Y., Luo, Y.F. & Gao, W. "Biosynthesis of paclitaxel using synthetic biology", Phytochem Rev (2021). https://doi.org/10.1007/s11101-021-09766-0 that elucidating the complete biosynthetic pathway of paclitaxel and realizing the industrialization of paclitaxel biosynthesis still requires a lot of work.

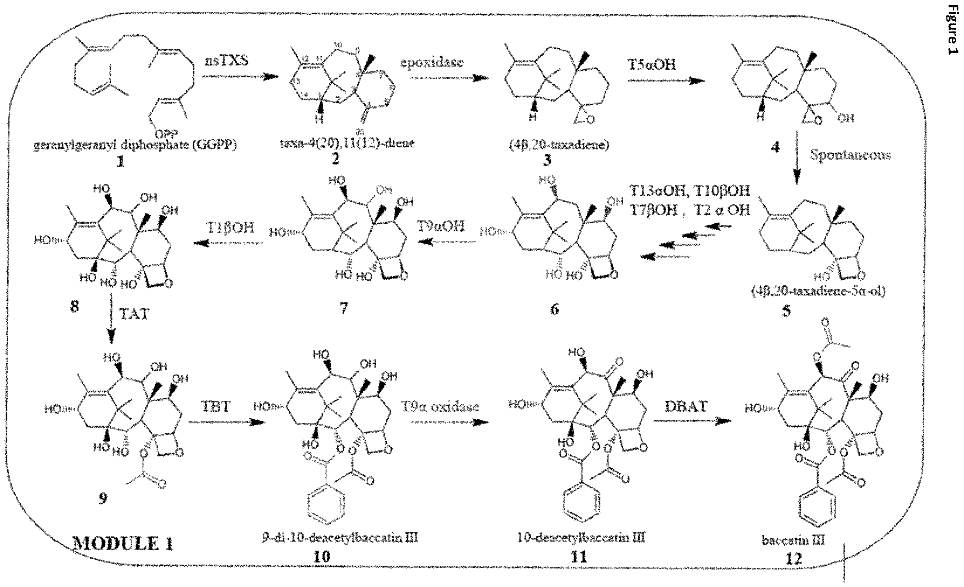

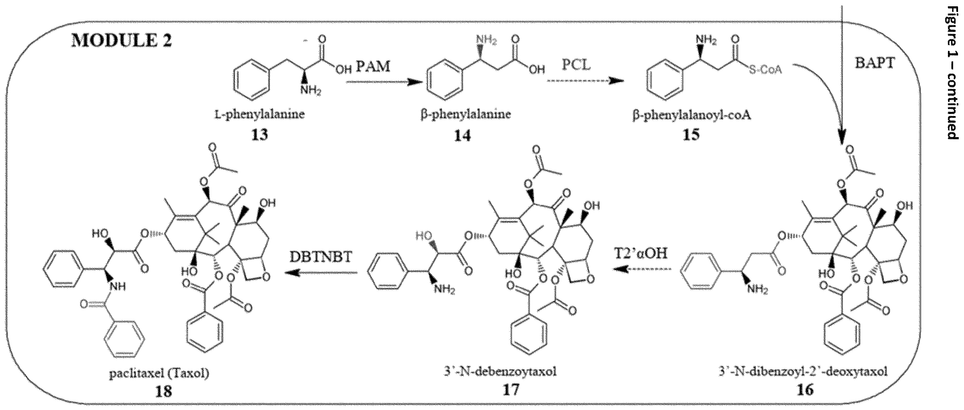

The present invention of the first times solves this obstacle by revealing the full pathway for paclitaxel biosynthesis. This has been achieved by integrating transcriptomics, cell biology, and metabolite profiling data (Fig.1 and examples section herein below).

Five missing enzymatic steps and one spontaneous conversion - as contrasted to the additional enzymatic steps as proposed by the prior art - were identified thereby elucidating the entire paclitaxel biosynthesis pathway. The activity of all the missing enzymes was confirmed by heterologous expression in *Nicotiana benthamiana.* Notably, the novel enzyme C4β-C20 epoxidase catalyzes the peculiar ring expansion reaction which could overcome the first bottleneck of metabolic engineering. Both, formerly characterized genes of the paclitaxel biosynthesis pathway as well as the newly identified genes of the paclitaxel biosynthesis pathway of the invention were used for successfully biosynthesizing two important intermediates of the paclitaxel biosynthesis pathway; 10-deacetylbaccatin III and baccatin III. Overall, the present invention establishes for the first time the metabolic route to taxoid biosynthesis in Taxus.

The present invention therefore relates in a first aspect to a method for producing 4β,20-taxadiene comprising (a) enzymatically converting taxa-4(5),11(12)-diene with a C4β,C20-epoxidase selected from (i) a polypeptide comprising or consisting of the amino acid sequence of SEQ ID NO: 3; (ii) a polypeptide being encoded by a nucleic acid molecule comprising or consisting of the nucleotide sequence of SEQ ID NO: 4; (iii) a polypeptide having an amino acid sequence being at least 80% identical, preferably at least 90% and most preferably at least 95% identical to the amino acid sequence of SEQ ID NO: 3; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 4; and (v) a fragment of the polypeptide of any of (i) to (iv) comprising at least 200 amino acids thereby producing 4β,20-taxadiene.

The complete biosynthesis of taxol (Paclitaxel) from the starting compound geranylgeranyl diphosphatase (GGPP) is shown in Figure 1, including the five missing enzymatic steps (4β,20-epoxidase, T1β hydroxylase, T9α hydroxylase, T9α oxidase, and β-phenylalanine coenzyme A ligase (PCL) and ) that were elucidated in accordance with the present invention. Figure 1 also shows the side biosynthesis of L-phenylalanine to β-phenylalanine-coA, noting that in the pathway of the biosynthesis of taxol β-phenylalanine-coA reacts with one of the precursors of taxol, as will be described in more detail herein below. Figure 1 furthermore shows all enzymes that are involved as well as all intermediate compounds along the complete biosynthesis pathway that were stable, so that they can be detected and isolated as distinct compounds.

In more detail, the arrows in Figure 1 shows that the 16 reactions (15 enzymatic reactions and one spontaneous reaction) are needed in order to arrive from GGPP to taxol. The educts and products of the 16 reactions are designated as compounds 1 to 18 in Figure 1. In this respect the following Table 1 is provided:

**Table 1**

| **Reaction No.** | **Enzymatic conversion of** | **SEQ ID NOs of Enzymes (aa and nt)** |
|---|---|---|
| 1 to 2 | geranylgeranyl diphosphatase (GGPP) with a taxadien synthase (nsTXS, EC 4.2.3.17) thereby producing taxa-4(5),11(12)-diene | 1 and 2 |
| | | 45 and 46⁺ |
| | | 47 and 48⁺ |
| ***2 to 3*** | ***taxa-4(5),11(12)-diene with a* *C4β*,*C20-epoxidase (also called "epoxidase" herein) thereby producing 46,20-taxadiene*** | **3 and 4** |
| 3 to 4; 4 to 5 | 4β,20-taxadiene with a T5αOH hydroxylase (EC 1.14.14.176) thereby producing a 5α hydroxylated intermediate and via a further spontaneous formation of the oxetane ring 4β,20-taxadiene-5α-ol | 5 and 6 |
| 5 to 6 | 4β,20-taxadiene-5α-ol with a T2αOH hydroxylase, a T7βOH hydroxylase (EC 1.14.13.147), a T10βOH hydroxylase (EC 1.14.13.76) and a T13αOH hydroxylase | 7 and 8 (T2) |
| | | 9 and 10 (T7) |
| | | 11 and 12 (T10) |
| | | 13 and 14 (T13) |
| | (EC 1.14.13.77) thereby producing 4β,20-taxadiene-2α,5α,7β,10β,13α-οl | |
| ***6 to 7*** | **4β,20-taxadiene-2α,5α,7β,10β,13α-ol *with a T9αOH hydroxylase thereby producing* 4β,20-taxadiene-2α,5α,7β,9α,10β,13α-ol** | **15 and 16** |
| ***7 to 8*** | ***4β,20-taxadiene-2α,5α,7β,9α,10β,13α-ol with α T1βOH hydroxylase thereby producing 46,20-taxadiene-1β,2α,5α,7β,9α,10β,13α-ol*** | **17 and 18** |
| 8 to 9 | 4β,20-taxadiene-1β,2α,5α,7β,9α,10β,13α-ol with taxadien-5α-ol-O-acetyl-transferase (TAT, EC 2.3.1.162) thereby producing 4-acetyloxy-4β,20-taxadiene-1β,2α,5α,7β,9α,10β,13α-ol | 19 and 20 |
| 9 to 10 | 4-acetyloxy-4β,20-taxadiene-1β,2α,5α,7β,9α,10β,13α-ol with taxane-2α-O-benzoyltransferase (TBT, EC 2.3.1.166) thereby producing 9-di-10-deacetylbaccatin III | 21 and 22 |
| ***10 to 11*** | ***9-di-10-deacetylbaccatin III with T9-oxidase thereby producing 10-deacetylbaccatin III*** | **25 and 26** |
| | | **39 and 40^{∗}** |
| | | **41 and 42^{∗}** |
| | | **43 and 44^{∗}** |
| 11 to 12 | 10-deacetylbaccatin III with 10-deacetylbaccatin III-10-O-acatyltransferase (DBAT, EC 2.3.1.167) thereby producing baccatin III | 27 and 28 |
| 12&15 to 16 | baccatin III and β-phenylalanoyl-coA with a baccatin III 13-O-(3-amino-3-phenylpropanoyl) transferase (BAPT, EC 2.3.1.B16) thereby producing 3'-N-debenzoyl-2'-deoxytaxol | 29 and 30 |
| 13 to 14 | L-phenylalanine with a phenylalanine aminomutase (PAM) thereby producing β-phenylalanine | 31 and 32 |
| ***14 to 15*** | ***β-phenylalanine with α β-phenylalanine coenzyme A ligase (PCL, EC 6.2.1.30) thereby producing β-phenylalanoyl-coA*** | **33 and 34** |
| 16 to 17 | enzymatically converting 3'-N-debenzoyl-2'-deoxytaxol with a T2'αOH hydroxylase thereby producing 3'-N-debenzoyltaxol | 35 and 36 |
| 17 to 18 | 3'-N-debenzoyltaxol with 3'-N-debenzoyltaxol-2'-deoxytaxol-N-benzoyltransferase (DBTNBT) thereby producing taxol (Paclitaxel) | 37 and 38 |

| | | |
|---|---|---|
| + SEQ ID NOs 45 and 46 is truncated nsTXS1 and SEQ ID NOs 47 and 48 is truncated nsTXS2. These variants of TXS are known to improve taxadien yields; see examples herein below. ^{∗} For the T9α-oxidase four different variants were identified. The variant of SEQ ID NOs 25 and 26 is preferred. | | |

The above Table 1 shows for all sixteen reactions, the enzymes (underlined) as well as the educts (or substrates) and the products of the 15 enzymatic conversions and the one spontaneous conversion. The conversions being shown in bold and italic in Table 1 as well as the spontaneous conversion are believed to be identified for the first time herein. The reactions are also shown in Figure 1. Hence, the enzymatic activity of each of the involved enzymes can be determined by determining their capability to catalyze their respective enzymatic conversions. The enzyme/enzymes is/are enzymatically active if upon the contact of the enzyme/enzymes and the educt under suitable conditions (for example, within a cell) the formation of the product can be detected. Means and methods for the detection of the product are known in the art. Preferably a mass spectrometric analysis is used and most preferably gas chromatography-mass spectrometric (GC-MS) analysis and/or ultra-high-performance liquid chromatography high-resolution mass spectrometry (UHPLC-HRMS) as illustrated in the appended examples. Means and methods for quantifying enzymatic activity will be discussed herein below.

The enzymes in Figure 1 have the following basic activities:
1) Taxadien synthase is a carbon-oxygen lyase acting on phosphatases. The cyclization being catalyzed by this enzyme involves a 1,5-hydride shift of geranylgeranyl-diphosphate diphosphate.
2) C4β,C20-epoxidase is an epoxidase. This epoxidase is an enzyme being capable of forming an epoxide between C4 and C20, which is a cyclic ether with a three-atom ring.
3) T5αOH (EC 1.14.14.176) hydroxylase is an oxidoreductase. It is acting on paired donors, with incorporation or reduction of molecular oxygen and with reduced flavin or flavoprotein as one donor, and incorporation of one atom of oxygen into the other donor. Once position 5 is hydroxylated this -OH group and the neighbouring cyclic ether with a three-atom ring spontaneously form an oxetane ring.
4) The enzymes T2αOH hydroxylase, T7βOH hydroxylase (EC 1.14.13.147), T10βOH hydroxylase (EC 1.14.13.76) and T13αOH hydroxylase (EC 1.14.13.77) are all oxidoreductases. They act on paired donors, with incorporation or reduction of molecular oxygen, and with NADH or NADPH as one donor, and incorporation of one atom of oxygen into the other donor.
5) T9αOH hydroxylase is an oxidoreductase. It acts on paired donors, with incorporation or reduction of molecular oxygen, and with NADH or NADPH as one donor, and incorporation of one atom of oxygen into the other donor.
6) T1βOH hydroxylase is an oxidoreductase. It acts on paired donors, with incorporation or reduction of molecular oxygen.
7) taxadien-5α-ol-O-acetyl-transferase (TAT, EC 2.3.1.162) is an acyltransferase transferring groups other than aminoacyl groups.
8) taxane-2α-O-benzoyltransferase (TBT, EC 2.3.1.166) is an acyltransferase transferring groups other than aminoacyl groups.
9) T9α-oxidase is an oxidase. Oxidases are enzymes that catalyze oxidation-reduction reactions, especially one involving dioxygen (O₂) as the electron acceptor.
10) 10-deacetylbaccatin III-10-O-acatyltransferase (DBAT, EC 2.3.1.167) is an acyltransferase transferring groups other than aminoacyl groups.
11) 13-O-(3-amino-3-phenylpropanoyl) transferase (BAPT, EC 2.3.1.B16) is also an acyltransferase transferring groups other than aminoacyl groups.
12) phenylalanine aminomutase (PAM) is an intramolecular transferase that transfers amino groups.
13) β-phenylalanine coenzyme A ligase (PCL, EC 6.2.1.30) is a ligase, in particular an acid-thiol ligase that forms carbon-sulfur bonds;
14) T2'αOH hydroxylase is an oxidoreductase. It acts on paired donors, with incorporation or reduction of molecular oxygen.
15) 3'-N-debenzoyltaxol-2'-deoxytaxol-N-benzoyltransferase (DBTNBT) is a transferase. Transferases catalyse the transfer of specific functional groups (e.g. a methyl or glycosyl group) from one molecule (called the donor) to another (called the acceptor).

As mentioned, the five novel enzymatic conversions are shown in *italic* and **bold** in the above Table 1. In connection with each of the five novel enzymatic conversions also a novel enzyme was revealed. In this respect it is noted that amino acid and nucleotide sequences of the enzymes were known from genome databases. However, the functions and the enzymatic conversions of these enzymes are revealed and characterized for the first time herein.

The first aspect of the invention relates to the novel enzymatic conversion 2 to 3 of Table 1 that is catalyzed by the novel enzyme C4β,C20-epoxidase (also called "epoxidase" herein). As discussed above, the peculiar ring expansion reaction as catalyzed by this enzyme could overcome the first bottleneck of metabolic engineering and thereby significantly facilitate the recombinant production of taxol as well as any desired precursor thereof, such as baccatin III.

In this respect it is also of note that the five novel enzymatic conversions as identified herein form part of the entire biosynthesis pathway of taxol but that the use of these enzymatic conversions is not limited to this particular pathway. This is because each of the five novel enzymatic conversions enables the production of a specific precursor of taxol already as such being of commercial value. In addition, the follow-up reactions may be used in order to produce taxol derivatives or the follow-up reactions may be different than the above reactions in Table 1.

In accordance with a preferred embodiment of the first aspect of the invention the method further comprises prior to step (a) (a') enzymatically converting geranylgeranyl pyrophosphate (GGPP) with a taxadien synthase (EC 4.2.3.17) thereby producing texa-4(5),11(12)-diene, preferably wherein the taxadien synthase is selected from (i) a polypeptide comprising or consisting of the amino acid sequence of SEQ ID NO: 1; (ii) a polypeptide being encoded by a nucleic acid molecule comprising or consisting of the nucleotide sequence of SEQ ID NO: 2; (iii) a polypeptide having an amino acid sequence being at least 80% identical, preferably at least 90% and most preferably at least 95% identical to the amino acid sequence of SEQ ID NO: 1; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 2; and (v) a fragment of the polypeptide of any of (i) to (iv) comprising at least 600 amino acids.

This preferred embodiment of the first aspect of the invention relates to the enzymatic conversion 1 to 2 of Table 1 that is catalyzed by the enzyme taxadien synthase (EC 4.2.3.17). The educt GGPP is produced through the methylerythritol phosphate pathway (MEP) that is known. Geranylgeranyl pyrophosphate ammonium salt is commercially available, for example, from Merck or Sigma.

SEQ ID NOs 45 and 46 and SEQ ID NOs 47 and 48 as well as a polypeptide having an amino acid sequence being at least 80% identical, preferably at least 90% and most preferably at least 95% identical to the amino acid sequence of SEQ ID NO: 45 or 47 and a polypeptide being encoded by a nucleic acid molecule comprising or consisting of a nucleotide sequence which is at least 80% identical, preferably at least 90% and most preferably at least 95% identical to the nucleotide sequence of SEQ ID NO: 46 and 48 are preferred examples of fragments of the polypeptide of any of (i) to (iv).

In accordance with a more preferred embodiment of the first aspect of the invention the method further comprises prior to steps (a) and (a') (a") enzymatically converting farnesyl diphosphate and isopentenyl diphosphate with a geranylgeranyl diphosphate synthase (GGPPS; EC 2.5.1.29) thereby producing geranylgeranyl diphosphate, preferably wherein the GGPPS is selected from (i) a polypeptide comprising or consisting of the amino acid sequence of SEQ ID NO: 23; (ii) a polypeptide being encoded by a nucleic acid molecule comprising or consisting of the nucleotide sequence of SEQ ID NO: 24; (iii) a polypeptide having an amino acid sequence being at least 80% identical, preferably at least 90% and most preferably at least 95% identical to the amino acid sequence of SEQ ID NO: 23; (iv)a polypeptide being encoded by a nucleic acid molecule comprising or consisting of a nucleotide sequence which is at least 80% identical, preferably at least 90% and most preferably at least 95% identical to the nucleotide sequence of SEQ ID NO: 24; and (v) a fragment of the polypeptide of any of (i) to (iv) comprising at least 300 amino acids.

This preferred embodiment of the first aspect of the invention relates to the production of GGPP via the enzyme of the methylerythritol phosphate pathway (MEP) geranylgeranyl diphosphate synthase (GGPPS; EC 2.5.1.29) from the educts farnesyl diphosphate and isopentenyl diphosphate. Farnesyl diphosphate is a natural product found in *Streptomyces albidoflavus, Streptomyces coelicolor,* and other organisms, and thus can be easily obtained. Isopentenyl diphosphate can be synthesised via an alternative non-mevalonate pathway of isoprenoid precursor biosynthesis, the MEP pathway, where it is formed from (E)-4-hydroxy-3-methyl-but-2-enyl pyrophosphate (HMB-PP) by the enzyme HMB-PP reductase (LytB, IspH). The MEP pathway is present in many bacteria, apicomplexan protozoa such as malaria parasites, and in the plastids of higher plants. Hence, also isopentenyl diphosphate can be easily obtained.

The present invention relates in a second aspect to a method for producing 10-deacetylbaccatin III comprising (a) enzymatically converting 9-di-10-deacetylbaccatin III with an T9α-oxidase selected from (i) a polypeptide comprising or consisting of the amino acid sequence of SEQ ID NO: 25, 39, 41 or 43; (ii) a polypeptide being encoded by a nucleic acid molecule comprising or consisting of the nucleotide sequence of SEQ ID NO: 26, 40, 42 or 44; (iii) a polypeptide having an amino acid sequence being at least 80% identical, preferably at least 90% and most preferably at least 95% identical to the amino acid sequence of SEQ ID NO: 25, 39, 41 or 43; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 26, 40, 42 or 44; and (v) a fragment of the polypeptide of any of (i) to (iv) comprising at least 300 amino acids thereby producing 10-deacetylbaccatin III.

The second aspect of the invention relates to the novel enzymatic conversion 10 toll in Table 1 that is catalyzed by the novel T9α-oxidase. The T9α-oxidase is an oxidase that modifies the -OH group at position 9 of the taxadiene chemical body. By the identification of the T9α-oxidase one of the key enzymes being involved in the taxol pathway has been identified. This is because this enzyme is responsible for the formation of the oxo group ring in 10-deacetylbaccatin III that is also found in baccatin III. 10-deacetylbaccatin III is the direct precursor of baccatin III. Hence, the identification of T9α-oxidase provides and alternative way for the synthesis of baccatin III. As discussed above, it is already possible now to produce taxol from baccatin III.

The present invention relates in a third aspect to a method for producing baccatin III comprising (a) producing 4β,20-taxadiene according to the method of claim 1or 2; (b) enzymatically converting 4β,20-taxadiene with a T5αOH hydroxylase (EC 1.14.14.176) thereby producing 4β,20-taxadiene-5α-ol; (c) enzymatically converting 4β,20-taxadiene-5α-ol with a T2αOH hydroxylase, a T7βOH hydroxylase (EC 1.14.13.147), a T10βOH hydroxylase (EC 1.14.13.76) and a T13αOH hydroxylase (EC 1.14.13.77) thereby producing 4β,20-taxadiene-2α,5α,7β,10β,13β-ol; (d) enzymatically converting 4β,20-taxadiene-2α,5α,7β,10β,13α-ol with a T9αOH hydroxylase thereby producing 4β,20-taxadiene-2α,5α,7β,9α,10β,13α-οl; (e) enzymatically converting 4β,20-taxadiene-2α,5α,7β,9α,10β,13α-ol with a T1βOH hydroxylase thereby producing 4β,20-taxadiene-1β,2α,5α,7β, 9α,10β,13α-ol; (f) enzymatically converting 4β,20-taxadiene-1β,2α,5α,7β, 9α,10β,13α-ol with taxadien-5α-ol-O-acetyl-transferase (TAT, EC 2.3.1.162) thereby producing 4-acetyloxy-4β,20-taxadiene-1β,2α,5α,7β,9α,10β,13α-ol; (g) enzymatically converting 4-acetyloxy-4β,20-taxadiene-1β,2α,5α,7β,9α,10β,13α-ol with taxane-2α-O-benzoyltransferase (TBT, EC 2.3.1.166) thereby producing 9-di-10-deacetylbaccatin III; (h) enzymatically converting 9-di-10-deacetylbaccatin III with T9α-oxidase thereby producing 10-deacetylbaccatin III, preferably according to the method of claim 3; and (i) enzymatically converting 10-deacetylbaccatin III with 10-deacetylbaccatin III-10-O-acatyltransferase (DBAT, EC 2.3.1.167) thereby producing baccatin III..

The third aspect of the invention relates to the entire biosynthesis pathway from GGPP to baccatin III by the enzymatic conversions of Module 1 of Figure 1, which involve the novel enzymatic conversions 2 to 3, 6 to 7, 7 to 8 and 10 to 11 of Table 1. Hence, it was necessary to elucidate a total of four novel enzymatic conversions in order to enable the third aspect of the invention.

Again, is it already possible now to produce taxol from baccatin III, so that baccatin III is a very important precursor of taxol. Whereas the semisynthesis of taxol from baccatin III was already patented in 1986 this still does not allow a large-scale taxol production because, much like taxol itself, baccatin III can so far be obtained only in small quantities. The present invention has the potential to overcome this bottleneck.

In accordance with a preferred embodiment of the third aspect of the invention one or more of (b') to (h') apply: (b') the TSaOH hydroxylase is selected from (i) a polypeptide comprising or consisting of the amino acid sequence of SEQ ID NO: 5; (ii) a polypeptide being encoded by a nucleic acid molecule comprising or consisting of the nucleotide sequence of SEQ ID NO: 6; (iii) a polypeptide having an amino acid sequence being at least 80% identical, preferably at least 90% and most preferably at least 95% identical to the amino acid sequence of SEQ ID NO: 5; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 6; and (v) a fragment of the polypeptide of any of (i) to (iv) comprising at least 300 amino acids; (c') the T2αOH hydroxylase is selected from (i) a polypeptide comprising or consisting of the amino acid sequence of SEQ ID NO: 7; (ii) a polypeptide being encoded by a nucleic acid molecule comprising or consisting of the nucleotide sequence of SEQ ID NO:8; (iii) a polypeptide having an amino acid sequence being at least 80% identical, preferably at least 90% and most preferably at least 95% identical to the amino acid sequence of SEQ ID NO: 7; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 8; and (v) a fragment of the polypeptide of any of (i) to (iv) comprising at least 300 amino acids; and/or the T7βOH hydroxylase is selected from (i) a polypeptide comprising or consisting of the amino acid sequence of SEQ ID NO: 9; (ii) a polypeptide being encoded by a nucleic acid molecule comprising or consisting of the nucleotide sequence of SEQ ID NO: 10; (iii) a polypeptide having an amino acid sequence being at least 80% identical, preferably at least 90% and most preferably at least 95% identical to the amino acid sequence of SEQ ID NO: 9; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 10; and (v) a fragment of the polypeptide of any of (i) to (iv) comprising at least 300 amino acids; and/or the T10βOH hydroxylase is selected from (i) a polypeptide comprising or consisting of the amino acid sequence of SEQ ID NO: 11; (ii) a polypeptide being encoded by a nucleic acid molecule comprising or consisting of the nucleotide sequence of SEQ ID NO:12; (iii) a polypeptide having an amino acid sequence being at least 80% identical, preferably at least 90% and most preferably at least 95% identical to the amino acid sequence of SEQ ID NO: 11; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 12; and (v) a fragment of the polypeptide of any of (i) to (iv) comprising at least 300 amino acids; and/or the T13αOH hydroxylase is selected from (i) a polypeptide comprising or consisting of the amino acid sequence of SEQ ID NO: 13; (ii) a polypeptide being encoded by a nucleic acid molecule comprising or consisting of the nucleotide sequence of SEQ ID NO: 14; (iii) a polypeptide having an amino acid sequence being at least 80% identical, preferably at least 90% and most preferably at least 95% identical to the amino acid sequence of SEQ ID NO: 13; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 14; and (v) a fragment of the polypeptide of any of (i) to (iv) comprising at least 300 amino acids; (d') the T9αOH hydroxylase is selected from (i) a polypeptide comprising or consisting of the amino acid sequence of SEQ ID NO: 15; (ii) a polypeptide being encoded by a nucleic acid molecule comprising or consisting of the nucleotide sequence of SEQ ID NO: 16; (iii) a polypeptide having an amino acid sequence being at least 80% identical, preferably at least 90% and most preferably at least 95% identical to the amino acid sequence of SEQ ID NO: 15; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 16; and (v) a fragment of the polypeptide of any of (i) to (iv) comprising at least 300 amino acids; (e') the T1βOH hydroxylase is selected from (i) a polypeptide comprising or consisting of the amino acid sequence of SEQ ID NO: 17; (ii) a polypeptide being encoded by a nucleic acid molecule comprising or consisting of the nucleotide sequence of SEQ ID NO: 18; (iii) a polypeptide having an amino acid sequence being at least 80% identical, preferably at least 90% and most preferably at least 95% identical to the amino acid sequence of SEQ ID NO: 17; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 18; and (v) a fragment of the polypeptide of any of (i) to (iv) comprising at least 300 amino acids; (f') the TAT is selected from (i) a polypeptide comprising or consisting of the amino acid sequence of SEQ ID NO: 19; (ii) a polypeptide being encoded by a nucleic acid molecule comprising or consisting of the nucleotide sequence of SEQ ID NO: 20; (iii) a polypeptide having an amino acid sequence being at least 80% identical, preferably at least 90% and most preferably at least 95% identical to the amino acid sequence of SEQ ID NO: 19; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 20; and (v) a fragment of the polypeptide of any of (i) to (iv) comprising at least 300 amino acids; (g') the TBT is selected from (i) a polypeptide comprising or consisting of the amino acid sequence of SEQ ID NO: 21; (ii) a polypeptide being encoded by a nucleic acid molecule comprising or consisting of the nucleotide sequence of SEQ ID NO: 22; (iii) a polypeptide having an amino acid sequence being at least 80% identical, preferably at least 90% and most preferably at least 95% identical to the amino acid sequence of SEQ ID NO: 21; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 22; and (v) a fragment of the polypeptide of any of (i) to (iv) comprising at least 300 amino acids; (h') the T9α-oxidase is selected from (i) a polypeptide comprising or consisting of the amino acid sequence of SEQ ID NO: 25, 39, 41 or 43; (ii) a polypeptide being encoded by a nucleic acid molecule comprising or consisting of the nucleotide sequence of SEQ ID NO: 26, 40, 42 or 44; (iii) a polypeptide having an amino acid sequence being at least 80% identical, preferably at least 90% and most preferably at least 95% identical to the amino acid sequence of SEQ ID NO: 25, 39, 41 or 43; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 26, 40, 42 or 44; and (v) a fragment of the polypeptide of any of (i) to (iv) comprising at least 300 amino acids; and (i') the DBAT is selected from (i) a polypeptide comprising or consisting of the amino acid sequence of SEQ ID NO: 27; (ii) a polypeptide being encoded by a nucleic acid molecule comprising or consisting of the nucleotide sequence of SEQ ID NO: 28; (iii) a polypeptide having an amino acid sequence being at least 80% identical, preferably at least 90% and most preferably at least 95% identical to the amino acid sequence of SEQ ID NO: 27; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 28; and (v) a fragment of the polypeptide of any of (i) to (iv) comprising at least 300 amino acids.

This preferred embodiment of the third aspect of the invention further defines the enzymes being involved in the enzymatic conversions of Module 1 of Table 1 by reference to their amino acid and nucleotide sequences. The term "one or more of (b') to (i')" is with increasing preference 2 or more, 3 or more, 4 or more, 5 or more, 6 or more, 7 or more or all 8 of (b') to (i'). It is furthermore preferred that the 2 or more, 3 or more, 4 or more comprise 2 or more, 3 or more or all 4 of the novel enzymes being involved in the novel enzymatic conversions 2 to 3, 6 to 7, 7 to 8 and 10 to 11 of Table 1.

The present invention relates in a fourth aspect to a method for producing β-phenylalanoyl-coA (i) from β-phenylalanine by β-phenylalanine coenzyme A ligase (PLC, EC 6.2.1.30), or (ii) from L-phenylalanine by phenylalanine aminomutase (PAM, EC 5.4.3.11) and β-phenylalanine coenzyme A ligase (EC 6.2.1.30), wherein the β-phenylalanine coenzyme A ligase is selected from (i) a polypeptide comprising or consisting of the amino acid sequence of SEQ ID NO: 33; (ii) a polypeptide being encoded by a nucleic acid molecule comprising or consisting of the nucleotide sequence of SEQ ID NO: 34; (iii) a polypeptide having an amino acid sequence being at least 80% identical, preferably at least 90% and most preferably at least 95% identical to the amino acid sequence of SEQ ID NO: 33; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 34; and (v) a fragment of the polypeptide of any of (i) to (iv) comprising at least 300 amino acids; and preferably wherein the phenylalanine aminomutase is selected from (i) a polypeptide comprising or consisting of the amino acid sequence of SEQ ID NO: 31; (ii) a polypeptide being encoded by a nucleic acid molecule comprising or consisting of the nucleotide sequence of SEQ ID NO: 32; (iii) a polypeptide having an amino acid sequence being at least 80% identical, preferably at least 90% and most preferably at least 95% identical to the amino acid sequence of SEQ ID NO: 31; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 32; and (v) a fragment of the polypeptide of any of (i) to (iv) comprising at least 500 amino acids.

The fourth aspect of the invention relates to the novel enzymatic conversion 14 to 15 in Table 1 that is catalyzed by the novel β-phenylalanine coenzyme A ligase (PLC, EC 6.2.1.30), and optionally further relates to the "upstream" enzymatic conversion 13 to 14 in Table 1 that is catalyzed by the phenylalanine aminomutase (PAM, EC 5.4.3.11). Also the identification of the novel enzymatic conversion 14 to 15 is an important contribution to the art because thereby the full side-pathway of the taxol biosynthesis has been revealed that leads to the precursor β-phenylalanoyl-coA. The enzymatic conversion 12+15 to 16 in Table 1 is the only step in the taxol biosynthesis where two educts are enzymatically converted into one product.

The present invention relates in a fifth aspect to a method for producing 3'-N-debenzoyl-2'-deoxytaxol comprising (a) producing baccatin III according to the third aspect of the invention and/or producing β-phenylalanoyl-coA according to the fourth aspect of the invention; and (b) enzymatically converting baccatin III and β-phenylalanoyl-coA with a baccatin III 13-O-(3-amino-3-phenylpropanoyl) transferase (BAPT, EC 2.3.1.B16), thereby producing 3'-N-debenzoyl-2'deoxytaxol, preferably wherein the BAPT is selected from (i) a polypeptide comprising or consisting of the amino acid sequence of SEQ ID NO: 29; (ii) a polypeptide being encoded by a nucleic acid molecule comprising or consisting of the nucleotide sequence of SEQ ID NO: 30; (iii) a polypeptide having an amino acid sequence being at least 80% identical, preferably at least 90% and most preferably at least 95% identical to the amino acid sequence of SEQ ID NO: 29; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 30; and (v) a fragment of the polypeptide of any of (i) to (iv) comprising at least 300 amino acids.

As discussed herein above, neither the part of the taxol biosynthesis pathway that leads to baccatin III nor the part of the taxol biosynthesis pathway that leads to β-phenylalanoyl-coA were fully known and are revealed herein for the first time. In the enzymatic conversion 12+15 to 16 in Table 1 baccatin III and β-phenylalanoyl-coA are enzymatically converted to 3'-N-debenzoyl-2'-deoxytaxol. Accordingly, the fifth aspect of the invention relates to the enzymatic conversion 12+15 to 16 in Table 1, wherein prior to said conversion baccatin III is produced according to the third aspect of the invention and/or β-phenylalanoyl-coA is produced according to the fourth aspect of the invention.

The present invention relates in a sixth aspect to a method for producing taxol comprising (a) producing 3'-N-debenzoyl-2'-deoxytaxol according to the fourth aspect of the invention; (b) enzymatically converting 3'-N-debenzoyl-2'-deoxytaxol with a T2'αOH hydroxylase thereby producing 3'-N-debenzoyltaxol, preferably wherein the T2'αOH hydroxylase is selected from (i) a polypeptide comprising or consisting of the amino acid sequence of SEQ ID NO: 35; (ii) a polypeptide being encoded by a nucleic acid molecule comprising or consisting of the nucleotide sequence of SEQ ID NO: 36; (iii) a polypeptide having an amino acid sequence being at least 80% identical, preferably at least 90% and most preferably at least 95% identical to the amino acid sequence of SEQ ID NO: 35; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 36; and (v) a fragment of the polypeptide of any of (i) to (iv) comprising at least 300 amino acids; and (c) enzymatically converting 3'-N-debenzoyltaxol with 3'-N-debenzoyltaxol-2'-deoxytaxol-N-benzoyltransferase (DBTNBT) thereby producing taxol, preferably wherein the DBTNBT is selected from (i) a polypeptide comprising or consisting of the amino acid sequence of SEQ ID NO: 37; (ii) a polypeptide being encoded by a nucleic acid molecule comprising or consisting of the nucleotide sequence of SEQ ID NO: 38; (iii) a polypeptide having an amino acid sequence being at least 80% identical, preferably at least 90% and most preferably at least 95% identical to the amino acid sequence of SEQ ID NO: 37; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 38; and (v) a fragment of the polypeptide of any of (i) to (iv) comprising at least 300 amino acids.

The sixth aspect of the invention is related to the fifth aspect of the invention. First 3'-N-debenzoyl-2'-deoxytaxol is to be produced according to the fifth aspect of the invention and then the enzymatic conversions 16 to 17 and 17 to 18 in Table 1 results in taxol. As discussed, above taxol is the desired product of the taxol biosynthesis pathway since it an antic-cancer drug and currently it is not possible to produce enough taxol in order to meet the clinical demand of taxol.

The invention also relates to the methods of (I) and (II):
**(I)** A method for producing 4β,20-taxadiene-2α,5α,7β,9α,10β,13α-ol comprising (a) enzymatically converting 4β,20-taxadiene-2α,5α,7β,10β,13α-ol with a T9αOH hydroxylase selected from (i) a polypeptide comprising or consisting of the amino acid sequence of SEQ ID NO: 15; (ii) a polypeptide being encoded by a nucleic acid molecule comprising or consisting of the nucleotide sequence of SEQ ID NO: 16; (iii) a polypeptide having an amino acid sequence being at least 80% identical, preferably at least 90% and most preferably at least 95% identical to the amino acid sequence of SEQ ID NO: 15; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 16; and (v) a fragment of the polypeptide of any of (i) to (iv) comprising at least 300 amino acids thereby producing 4β,20-taxadiene-2α,5α,7β,9α,10β,13α-οl.
**(II)** A method for producing 4β,20-taxadiene-1β,2α,5α,7β,9α,10β,13α-ol comprising (a) enzymatically converting 4β,20-taxadiene-2α,5α,7β,9α,10β,13α-ol with a T1βOH hydroxylase selected from (i) a polypeptide comprising or consisting of the amino acid sequence of SEQ ID NO: 18; (ii) a polypeptide being encoded by a nucleic acid molecule comprising or consisting of the nucleotide sequence of SEQ ID NO: 18; (iii) a polypeptide having an amino acid sequence being at least 80% identical, preferably at least 90% and most preferably at least 95% identical to the amino acid sequence of SEQ ID NO: 17; (iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 18; and (v) a fragment of the polypeptide of any of (i) to (iv) comprising at least 300 amino acids thereby producing 4β,20-taxadiene-1β,2α,5α,7β,9α,10β,13α-οl.

The above methods (I) and (II) relate to the enzymatic conversions 6 to 7 and 7 to 8 in Table 1, respectively. Also these enzymatic conversions according to the above methods (I) and (II) were identified for the first time herein.

The present invention relates in a seventh aspect to the use of a C4β,C20-epoxidase as defined in connection with the first aspect of the invention for producing 4β,20-taxadiene from texa-4(5),11(12)-diene.

The present invention relates in an eighth aspect to the use of a T9α-oxidase as defined in connection with the second aspect of the invention for producing 10-deacetylbaccatin III from 9-di-10-deacetylbaccatin III.

The present invention relates in a ninth aspect to the use of a β-phenylalanine coenzyme A ligase (PLC, EC 6.2.1.30) as defined in connection with the fourth aspect of the invention for producing β-phenylalanoyl-coA from β-phenylalanine.

The seventh, eighth and ninth aspect of the invention relate to uses corresponding to the enzymatic conversions 6 to 7 and 7 to 8 in Table 1, respectively.

Also described herein are the uses of (I) and (II):
**(I)** The use of a T9αOH hydroxylase as defined in connection with the method of (I) of the invention for producing 4β,20-taxadiene-2α,5α,7β,9α,10β,13α-ol from 4β,20-taxadiene-2α,5α,7β,10β,13α-ol.
**(II)** The use of a T1βOH hydroxylase as defined in connection with the method of (II) of the invention for producing 4β,20-taxadiene-1β,2α,5α,7β,9α,10β,13α-ol from 4β,20-taxadiene-2α,5α,7β,9α,10β,13α-ol.

The uses of (I) and (II) relate to uses corresponding to the enzymatic conversions of the methods of (I) and (II).

The present invention relates in a tenth aspect to a vector comprising (I) a nucleic acid molecule encoding a C4β,C20-epoxidase, which nucleic acid molecule is (a) a nucleic acid molecule encoding a polypeptide comprising or consisting of the amino acid sequence of SEQ ID NO: 3; (b) a nucleic acid molecule comprising or consisting of the nucleotide sequence of SEQ ID NO: 4; (c) a nucleic acid molecule comprising or consisting of a nucleic acid molecule encoding a polypeptide having the activity of a catalase the amino acid sequence of which is at least 80% identical, preferably at least 90% and most preferably at least 95% identical to the amino acid sequence of SEQ ID NO: 3; (d) a nucleic acid molecule encoding a polypeptide comprising or consisting of a nucleotide sequence which is at least 80% identical, preferably at least 90% and most preferably at least 95% identical to the nucleotide sequence of SEQ ID NO: 4; (e) a fragment of the nucleic acid molecule of any of (i) to (iv) comprising at least 600 nucleic acids; or (f) the nucleic acid sequence of any of (a) to (e) wherein T is U; and/or (II) a nucleic acid molecule encoding an T9α oxidase, which nucleic acid molecule is (a) a nucleic acid molecule encoding a polypeptide comprising or consisting of the amino acid sequence of SEQ ID NO: 25, 39, 41 and 43; (b) a nucleic acid molecule comprising or consisting of the nucleotide sequence of SEQ ID NO: 26, 40, 42 and 44; (c) a nucleic acid molecule comprising or consisting of a nucleic acid molecule encoding a polypeptide having the activity of a catalase the amino acid sequence of which is at least 80% identical, preferably at least 90% and most preferably at least 95% identical to the amino acid sequence of SEQ ID NO: 25, 39, 41 and 43; (d) a nucleic acid molecule encoding a polypeptide comprising or consisting of a nucleotide sequence which is at least 80% identical, preferably at least 90% and most preferably at least 95% identical to the nucleotide sequence of SEQ ID NO: 26, 40, 42 and 44; (e) a fragment of the nucleic acid molecule of any of (i) to (iv) comprising at least 900 nucleic acids, or (f) the nucleic acid sequence of any of (a) to (e) wherein T is U; and/or (III) a nucleic acid molecule encoding a β-phenylalanine coenzyme A ligase (PLC, EC 6.2.1.30), which nucleic acid molecule is (a) a nucleic acid molecule encoding a polypeptide comprising or consisting of the amino acid sequence of SEQ ID NO: 33; (b) a nucleic acid molecule comprising or consisting of the nucleotide sequence of SEQ ID NO: 34; (c) a nucleic acid molecule comprising or consisting of a nucleic acid molecule encoding a polypeptide having the activity of a catalase the amino acid sequence of which is at least 80% identical, preferably at least 90% and most preferably at least 95% identical to the amino acid sequence of SEQ ID NO: 33; (d) a nucleic acid molecule encoding a polypeptide comprising or consisting of a nucleotide sequence which is at least 80% identical, preferably at least 90% and most preferably at least 95% identical to the nucleotide sequence of SEQ ID NO: 34; (e) a fragment of the nucleic acid molecule of any of (i) to (iv) comprising at least 900 nucleic acids, or (f) the nucleic acid sequence of any of (a) to (e) wherein T is U.

The vector of the present invention comprises one or more nucleic acid molecules encoding the C4β,C20-epoxidase and/or T9α oxidase and/or the β-phenylalanine coenzyme A ligase that were revealed for the first time in connection with the present invention. The vector can be used for the recombinant production of the product of the enzymatic conversion as catalyzed by these enzymes or can introduced into a(n) organism, tissue, cell, or organelle in order to produce recombinant organism, tissue, cell, or organelle, as will be further detailed herein below.

In accordance with a preferred embodiment of the tenth aspect of the invention the vector further comprises one or more nucleic acid molecules encoding one or more of the additional enzymes as defined herein above other than the C4β,C20-epoxidase and/or T9α oxidaseand/or the β-phenylalanine coenzyme A ligase (PLC, EC 6.2.1.30).

In this connection it is preferred that the one or more of the additional enzymes result in a vector or more than one vector that comprise(s) all enzymes of the taxol biosynthesis pathway as described herein that are required to produce baccatin III from GGPP (conversions 1 to 11 in Table 1) and/or the two enzymes of the taxol biosynthesis pathway as described herein that are required to produce β-phenylalanoyl-coA (conversions 12a and 12b in Table 1). Most preferably the vector or more than one vector comprise(s) all enzymes of the taxol biosynthesis pathway.

The present invention relates in a eleventh aspect to a recombinant organism, a tissue, a cell, or an organelle (i) recombinantly expressing the C4β,C20-epoxidase as defined in the first aspect of the invention and/or the T9α oxidase as defined in the second aspect of the invention and/or the β-phenylalanine coenzyme A ligase (PLC, EC 6.2.1.30) as defined in of the fourth aspect of the invention and optionally expressing one or more of the additional enzymes as defined herein above, (ii) comprising the nucleic acid molecule encoding a C4β,C20-epoxidase as defined in the tenth aspect of the invention and/or the nucleic acid molecule encoding an T9α oxidase as defined in of the tenth aspect of the invention and/or the nucleic acid molecule encoding an β-phenylalanine coenzyme A ligase (PLC, EC 6.2.1.30) as defined in of the tenth aspect of the invention, and optionally comprising one or more nucleic acid molecules encoding one or more of the additional enzymes as defined herein above, or (iii) carrying the vector of the tenth aspect of the invention.

Also for the eleventh aspect of the invention it is preferred that the one or more of the additional enzymes result in all enzymes of the taxol biosynthesis pathway as described herein that are required to produce baccatin III from GGPP (conversions 1 to 11 in Table 1) and/or the two enzymes of the taxol biosynthesis pathway as described herein that are required to produce β-phenylalanoyl-coA (conversions 12a and 12b in Table 1). Most preferably all enzymes of the taxol biosynthesis pathway are comprised in the recombinant organism, a tissue, a cell, or an organelle of the eleventh aspect of the invention.

In accordance with a preferred embodiment of the eleventh aspect of the invention the organism is a plant, fungus, or a bacterium.

The term "taxol" is used herein interchangeably with the term Paclitaxel. Paclitaxel, sold under the brand name Taxol among others, is used as a chemotherapy medication used to treat a number of types of cancer. The formula of taxol is C₄₇H₅₁NO₁₄ and its molecular weight is 853.918 g·mol⁻¹. Taxol also has the chemical names (see CAS Registry Number 33069-62-4):
- Benzenepropanoic acid, β-(benzoylamino)-α-hydroxy-, (2a*R*,4*S*,4a*S*,6*R*,9*S*,11*S*,12*S*,12a*R*,12b*S*)-6,12b-bis(acetyloxy)-12-(benzoyloxy)-2a,3,4,4a,5,6,9,10,11,12,12a,12b-dodecahydro-4,11-dihydroxy-4a,8,13,13-tetramethyl-5-oxo-7,11-methano-1*H*-cyclodeca[3,4]benz[1,2-*b*]oxet-9-yl ester, (α*R*,β*S*)-
- Tax-11-en-9-one, 5β,20-epoxy-1,2α,4,7β,10β,13α-hexahydroxy-, 4,10-diacetate 2-benzoate 13-ester with (2*R*,3*S*)-*N*-benzoyl-3-phenylisoserine
- Benzenepropanoic acid, β-(benzoylamino)-α-hydroxy-, 6,12b-bis(acetyloxy)-12-(benzoyloxy)-2a,3,4,4a,5,6,9,10,11,12,12a,12b-dodecahydro-4,11-dihydroxy-4a,8,13,13-tetramethyl-5-oxo-7,11-methano-1*H*-cyclodeca[3,4]benz[1,2-*b*]oxet-9-yl ester, [2a*R-*[2aα,4β,4aβ,6β,9α(α*R*^{∗},β*S*^{∗}),11α,12α,12aα,12bα]]-
- 7,11-Methano-1*H*-cyclodeca[3,4]benz[1,2-*b*]oxete, benzenepropanoic acid derivative

The term "polypeptide" as used herein is used interchangeably with the term protein and refers to polymers constructed from one or more chains of amino acid residues linked by peptide bonds. The term "polypeptides" also refers to chemically or post-translationally modified polypeptides. Generally, the terms apply to naturally occurring amino acid polymers, as well as to amino acid polymers in which one or more amino acid residues are non-naturally occurring amino acids, such as artificial chemical analogues of naturally-occurring amino acids.

The term "nucleic acid molecule" in accordance with the present invention includes DNA, such as cDNA or double or single stranded genomic DNA and RNA. In this regard, "DNA" (deoxyribonucleic acid) means any chain or sequence of the chemical building blocks adenine (A), guanine (G), cytosine (C) and thymine (T), called nucleotide bases, that are linked together on a deoxyribose sugar backbone. DNA can have one strand of nucleotide bases, or two complimentary strands which may form a double helix structure. "RNA" (ribonucleic acid) means any chain or sequence of the chemical building blocks adenine (A), guanine (G), cytosine (C) and uracil (U), called nucleotide bases, that are linked together on a ribose sugar backbone. RNA typically has one strand of nucleotide bases, such as mRNA. Included are also single- and double-stranded hybrids molecules, i.e., DNA-DNA, DNA-RNA and RNA-RNA. The nucleic acid molecule may also be modified by many means known in the art. Non-limiting examples of such modifications include methylation, "caps", substitution of one or more of the naturally occurring nucleotides with an analog, and internucleotide modifications such as, for example, those with uncharged linkages (e.g., methyl phosphonates, phosphotriesters, phosphoroamidates, carbamates, etc.) and with charged linkages (e.g., phosphorothioates, phosphorodithioates, etc.). Nucleic acid molecules, in the following also referred as polynucleotides, may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. The polynucleotides may be derivatized by formation of a methyl or ethyl phosphotriester or an alkyl phosphoramidate linkage. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA), peptide nucleic acid (PNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. Also included are nucleic acids containing modified bases, for example thio-uracil, thio-guanine and fluoro-uracil. A nucleic acid molecule typically carries genetic information, including the information used by cellular machinery to make proteins and/or polypeptides. The nucleic acid molecule according to the invention may additionally comprise promoters, enhancers, response elements, signal sequences, polyadenylation sequences, introns, 5'- and 3'- non-coding regions, and the like.

The method of producing taxol and intermediates thereof as described herein may also be characterized as methods for the enzymatic conversion of respective educts of taxol and intermediates thereof. This is because all reactions of the taxol pathway are all enzymatic conversions. Enzymatic conversions are biological/biochemical processes that may also occur ex vivo but preferably occur in a cell or organisms, such as am animal, plant or microorganism.

Enzymes are proteins that act as biological catalysts (biocatalysts). Catalysts accelerate chemical reactions. The molecules upon which enzymes may act are called substrates or more generally educt, and the enzyme converts the substrates into different molecules known as products.

In accordance with the present invention, the term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g. 80%, 85%, 90% or 95% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Nucleotide and amino acid sequence analysis and alignment in connection with the present invention are preferably carried out using the NCBI BLAST algorithm (Stephen F. Altschul, Thomas L. Madden, Alejandro A. Schäffer, Jinghui Zhang, Zheng Zhang, Webb Miller, and David J. Lipman (1997), Nucleic Acids Res. 25:3389-3402). BLAST can be used for nucleotide sequences (nucleotide BLAST) and amino acid sequences (protein BLAST). The skilled person is aware of additional suitable programs to align nucleic acid sequences.

As defined herein, sequence identities of at least 80% identical, preferably at least 90% identical, and most preferred at least 95% are envisaged by the invention. However, also envisaged by the invention are with increasing preference sequence identities of at least 97.5%, at least 98.5%, at least 99%, at least 99.5%, at least 99.8%, and 100%.

The polypeptides of items (iii) and (iv) of the various embodiments describes herein above that are defined in terms of sequence identity as well as the fragments that are defined by reference to certain length of the polypeptides of items (i) to (iv) are all required to retain the enzymatic activity of the respective base enzyme of the taxol pathway. For instance, in connection with the first aspect the polypeptides of items (iii) and (iv) as well as the fragments of item (v) are a C4β,C20-epoxidase which means the they are an enzyme being capable of enzymatically converting taxa-4(5),11(12)-diene to produce 4β,20-eposytax-11-ene. Means and methods for the detection the product, of an enzymatic reaction, such as produce 4β,20-eposytax-11-ene have been described herein above.

The polypeptides of items (iii) and (iv) as well as the fragments of item (v) maintain with increasing preference at least 50%, at least 60%, at least 70%, at least 80%, at least 90%, at least 95%, and 100% of the enzymatic catalytic activity of the corresponding based enzyme as it occurs nature. The enzyme's catalytic activity is preferably measures as enzyme unit (symbol U). 1U (µmol/min) is defined as the amount of the enzyme that catalyzes the conversion of one micromole of substrate per minute under the specified conditions of the assay method. The specified conditions are usually the optimum conditions, which include but are not limited to temperature, pH, and substrate concentration, that yield the maximal substrate conversion rate for that particular enzyme. The temperature is preferably 25°C.

The fragment lengths are with increasing preference for each of the above SEQ ID NOs independently at least 250, at least 300, at least 350, at least 400, at least 450, at least 500, at least 550, at least 600, at least 650, at least 700, at least 750, at least 800, and at least 850 amino acids. Accordingly, the fragments lengths are also with increasing preference at least 750, at least 900, at least 1050, at least 1200, at least 1350, at least 1500, at least 1650, at least 1800, at least 1950, at least 2100, at least 2250, at least 2400, at least 2550, at least 2700, and at least 2850 nucleotides. For each SEQ ID NO: suitable fragment length is selected. For instance, fragments of SEQ ID NO: 1 (862 amin acids) of at least 600 amino acids are envisioned herein above, so that with increasing preference fragments of SEQ ID NO: 1 of at least 650 amino acids, at least 700 amino acids, at least 750 amino acids, at least 800 amino acids and at least 850 amino acids are envisioned herein. As further example it is noted that fragments of SEQ ID NO: 3 (274 amin acids) of at least 200 amino acids are envisioned herein above, so that fragments of SEQ ID NO: 2 of at least 250 amino acids are preferred.

Also described herein are fragments that are with increasing preference 50 or less, 40 or less, 30 or less, or 20 or less, or 10 or less, or 5 or less amino shorter than the amino acid sequences of SEQ ID NOs 1 to 48 (i.e. the odd numbers 1, 3, 4, etc.). Similarly, also described herein are fragments that are with increasing preference 150 or less, 120 or less, 90 or less, or 60 or less, or 30 or less, or 15 or less nucleotides shorter than the nucleotide sequences of SEQ ID NOs 1 to 48 (i.e. the even numbers 2, 4, 6, etc.).

The fragments that are defined herein above by reference to a certain length of the polypeptides of items (i) to (iv) are preferably fragments that are defined by reference to a certain length of the polypeptides of items (i) and (ii).

The term "vector" in accordance with the invention means preferably a plasmid, cosmid, virus, bacteriophage or another vector used e.g. conventionally in genetic engineering which carries the nucleic acid molecule of the invention. The nucleic acid molecule of the invention may, for example, be inserted into several commercially available vectors. Non-limiting examples include prokaryotic plasmid vectors, such as of the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for Pichia pastoris comprise e.g. the plasmids pAO815, pPIC9K and pPIC3.5K (all Invitrogen).

The nucleic acid molecules inserted into the vector can e.g. be synthesized by standard methods, or isolated from natural sources. Ligation of the coding sequences to transcriptional regulatory elements and/or to other amino acid encoding sequences can also be carried out using established methods. Transcriptional regulatory elements (parts of an expression cassette) ensuring expression in prokaryotes or eukaryotic cells are well known to those skilled in the art. These elements comprise regulatory sequences ensuring the initiation of transcription (e. g., translation initiation codon, promoters, such as naturally-associated or heterologous promoters and/or insulators; see above), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers. Preferably, the polynucleotide encoding the polypeptide/protein or fusion protein of the invention is operatively linked to such expression control sequences allowing expression in prokaryotes or eukaryotic cells. The vector may further comprise nucleic acid sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

Furthermore, it is preferred that the vector comprises a selectable marker. Examples of selectable markers include genes encoding resistance to neomycin, ampicillin, hygromycine, and kanamycin. Specifically-designed vectors allow the shuttling of DNA between different hosts, such as bacteria-fungal cells or bacteria-animal cells (e. g. the Gateway system available at Invitrogen). An expression vector according to this invention is capable of directing the replication, and the expression, of the polynucleotide and encoded peptide or fusion protein of this invention. Apart from introduction via vectors such as phage vectors or viral vectors (e.g. adenoviral, retroviral), the nucleic acid molecules as described herein above may be designed for direct introduction or for introduction via liposomes into a cell. Additionally, baculoviral systems or systems based on vaccinia virus or Semliki Forest virus can be used as eukaryotic expression systems for the nucleic acid molecules of the invention.

The term "recombinant" means that the organism, tissue, cell or organelle is "genetically engineered". A recombinant or genetically engineered organism, tissue, cell or organelle that recombinantly expresses at least one enzyme that is not expressed by a corresponding (wild-type) organism, tissue, cell or organelle that has been used to prepare the genetically engineered organism, tissue, cell or organelle. It follows that the genetically engineered organism, tissue, cell or organelle has been prepared by technical means and does not occur in nature. In this respect it is to be understood that the term "is not expressed" preferably means that no expression of the at least one enzyme is found in a corresponding (wild-type) organism, tissue, cell or organelle. However, the term "is not expressed" also comprises the situation where essentially no expression of the at least one enzyme is found in a corresponding (wild-type) organism, tissue, cell or organelle.

The organism is preferably a microorganism or a plant. A microorganism is an organism of microscopic or ultramicroscopic size that generally cannot be seen by the naked eye. Viruses are not classified as microorganisms in accordance with the present disclosure. Examples of microorganisms include bacteria, archaea, protozoa, fungi and algae. The microorganism is preferably a bacterium or fungus and is most preferably a bacterium, such as *E. coli.* The fungus is preferably yeast and is most preferably Saccharomyces cerevisiae. The plant is preferably a taxus plant, such as *Taxus chinensis* or *Taxus brevifolia.*

When providing or producing the organism, tissue, cell or organelle of the invention, the skilled person can readily rely on its common general knowledge and the teaching of the invention. In particular, respective means and methods for genetically engineering and transfecting/transforming, respectively are known in the art and are, for example, disclosed in WO 2011/099006, WO 03/100066, EP 2 093 283 and Shih et al., 2014 (*loc. cit.*)*.*

Both, a transiently transformed/transfected and stably transformed/transfected organism, tissue, cell or organelle is encompassed by this invention. A stably transformed/transfected transgenic organism, tissue, cell or organelle is particularly preferred. In a preferred embodiment, the transgenic organism is a fertile transgenic plant from which seed can be harvested, and the invention further claims transgenic seeds of such a transgenic plant.

The transgenic tissue, cell or organelle or transgenic organism of the invention can be obtained by use of any appropriate transient or stable, integrative or non-integrative transformation method known in the art or presently disclosed. The respective nucleotide sequences (e.g. recombinant DNA constructs) can in principle be transcribed in any cell, tissue or organelle or in a whole organism of any developmental stage. However, the herein elsewhere described compartimentation may be advantageous also in this context,

The nucleotide sequence(s) encoding the enzyme(s) in accordance with the invention may be introduced into the organism's, tissue's, cell's or organelle's genome (where the organelle has a genome). The enzyme(s) may be expressed from this genome, for example from the mentioned introduced nucleotide sequence(s).

For example, the organelle may be genetically engineered so as to produce/express the enzyme(s) (see Bock R., 2014, Curr. Opin. Biotechnol. 26, 7-13 for genetical engineering of plastids). The enzyme(s) may be transcribed from the organelle's genome (where the organelle has a genome), for example from an encoding nucleotide sequence introduced therein (e.g. recombinant DNA construct). The organelle may comprise a nucleotide sequence which encodes and expresses the enzyme(s).

It is envisaged in the context that the enzymes may either be expressed directly in the organelle (e.g in the plastid and in particular in the plastid's stroma (matrix)) or may be targeted into the organelle (e.g in the plastid and in particular into the plastid's stroma (matrix)), e.g. after transcription/translation in the nucleus/cytosol.

In a preferred embodiment, the organelle to be comprised in the organism/tissue/cell of the invention, and the organelle of the invention, respectively, in particular the organelle which comprises (expresses) the enzyme(s) to be employed in accordance with the invention is a chloroplast. However, for example by choosing appropriate expression signals, it is also possible to express plastid transgenes encoding the enzyme(s) in non-green tissues, i.e. in other types of plastids (cf. Zhang, Plant J. 72 (2012) 115-128; Caroca, Plant J. 73 (2013) 368-379). Such other types of plastids are described herein elsewhere. For example, the enzyme(s) may be expressed from the mentioned nucleotide sequence(s) and/or from the plastids genome, respectively.

The skilled person is readily able to target (an) enzyme(s) in accordance with the invention into the relevant cell organelle/compartment. Thereby, the skilled person can, for example, rely on Emanuelsson (Nature Protocols 2, 953-971 2007), WO 2011/099006, WO 03/100066, EP 2 093 283 and Shih (loc. cit.). In particular, targeting domains for the desired cell organelles may be used and may, hence, be comprised in the nucleotide sequences/constructs described herein. The respective enzyme may then be translated as a respective precursor with an amino (N)-terminal extension (the targeting domain). Usually, in case the enzymes are to be targeted into the plastid, mitochondria or peroxisome, transit peptides (TP), pre-sequences or peroxisomal targeting signals (e.g. PTS1 and PTS2) are used as targeting domains, respectively.

In particular, if the enzymes are to be targeted into the plastid, N-terminal extensions acting as the "zip code" (also called "transit peptides") may be used. The targeting domains for plastids may be such which are recognized by Toes (proteins of the outer membrane complex which shuttles the pre-protein through the outer membrane) and/or Tics (proteins of the inner membrane complex which shuttles the pre-protein through the inner membrane). The (pre-)proteins/enzymes may further be assembled with chaperons. In a preferred aspect the enzymes may be stroma (matrix)-targeted enzymes. Respective nucleic acid constructs may comprise stroma (matrix)-targeting domains.

The skilled person is further readily able to provide an organism (a plant) which comprises/expresses the enzyme(s) to be employed in accordance with the invention in certain plastids (e.g. in chloroplasts), and, optionally, not to comprise/express the enzyme(s) in other plastids (e.g. amyloplasts), as the case may be. For example, such a selective expression/production of the enzyme(s) in the respective plastids can readily be achieved by the choice of, for example, (a) respective suitable element(s) like (a) promoter(s) and/or (a) signaling sequence(s) (targeting domain(s)), and the like.

Means and methods to genetically engineer an organism, tissue, cell (e.g. a plant) and/or a plastid are well known in the art and are, for example, described in Valkov, Transgenic Res. 20, 137 (2011) and Svab, Proc. Natl. Acad. Sci. USA 90, 913 (1993), WO 2011/099006, WO 2003/100066, EP 2 093 283, Shih et al. 2014 (loc. cit.) and Bock R., Curr. Opin. Biotechnol. 26, 7-13 (2014). An example of such a method is biolistic transformation (particle bombardment), for example with gold particles coated with the nucleotide sequence (s) (e.g. recombinant DNA construct) encoding the enzyme(s) in accordance with the invention. The respective means may, for example be a PDS1000/He particle delivery system, for example equipped with a Hepta adaptor (BioRad, Hercules, CA, USA).

Where a nucleotide sequence (e.g. recombinant DNA construct) is used to produce a (genetically engineered transgenic, e.g. transplastomic, organism (e.g. plant, or transgenic, e.g. transplastomic, tissue, cell or plastid thereof, of this invention), genetic engineering, transfection and transformation, respectively, can include any of the well-known and demonstrated methods and compositions. Suitable methods for, for example, plant transformation include virtually any method by which DNA can be introduced into a (plant) cell, in particular into a plastid, such as by direct delivery of DNA (e. g., by PEG-mediated transformation of protoplasts, by electroporation, by agitation with silicon carbide fibers, and by acceleration of DNA coated particles), by *Agrobacterium*-mediated transformation, by viral or other vectors, etc. One preferred method of plant transformation is microprojectile bombardment, for example, as illustrated in U.S. Patents 5,015,580 (soy), 5,550,318 (maize), 5,538,880 (maize), 6,153,812 (wheat), 6,160,208 (maize), 6,288,312 (rice) and 6,399,861 (maize), and 6,403,865 (maize).

In particular, the mRNA encoding the enzyme(s) may be expressed by transcription from a nucleotide sequence (for example DNA) following a promoter.

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a majority of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The following figure legends describe what the Figures show.
**Fig. 1****. The pathway of paclitaxel biosynthesis.** The catalytic sequence of seven hydrogenation steps is unclear. Black dotted lines represent newly identified steps. Non-black dotted lines represent previously identified steps. Module 1 represents represent baccatin III biosynthesis. Module 2 represents the second part of the pathway. TXS (taxadiene synthase), TSaOH (taxane 5α-hydroxylase), TAT (taxadiene-5α-ol-O-acetyl transferase), T10βOH (taxane 10β-hydroxylase), T13αOH (taxane 13α-hydroxylase), T2αOH (taxane 2α-hydroxylase), T9αOH (taxane 9α-hydroxylase), T7βOH (taxane 7β-hydroxylase), T1βOH (taxane 1β-hydroxylase), TBT (taxane-2α-O-benzoyltransferase), DBAT (10-deacetyl baccatin III-10-O-acetyltransferase), BAPT (baccatin III-3-amino, 13-phenylpropanoyltransferase), T2'αOH (taxane 2'α-hydroxylase), DBTNBT (3'-*N*-debenzoyl-2'-deoxytaxol-*N*-benzoyltransferase), PAM (phenylalanine aminomutase), PCL (β-phenylalanine coenzyme A ligase).

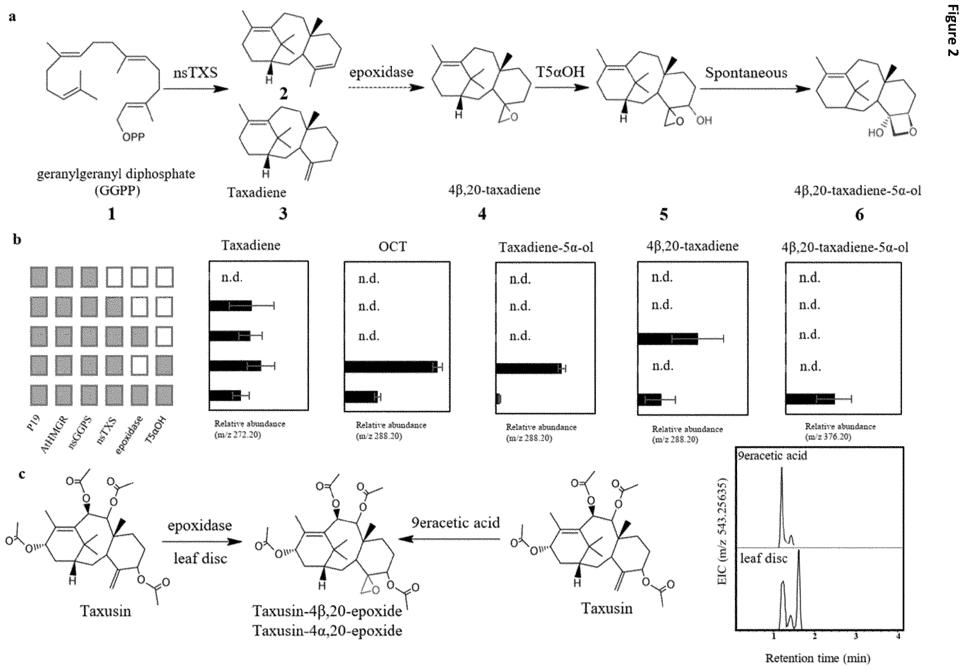

**Fig. 2****. Identification of taxadiene epoxidase.** a, Predicted first part of taxane biosynthesis. b, truncated GAPPS and TXS were co-expressed with C4β-C20-epoxidase or/and TSaOH. The relative abundance of taxadiene, OCT and taxadiene-5α-ol were measured by normal gas chromatography-mass spectrometry (GC-MS). Both 4β,20-taxadiene and 4β,20-taxadiene-5α-ol were measured by N,*O-*bis(trimethylsilyl)trifluoroacetamide (BSTFA) derivatized GC-MS. c, enzyme assay of epoxidase using taxusin as substrate. Both taxusin-4β,20-epoxide and taxusin-4α,20-epoxide were generated by either enzyme assay or chemical synthesis²⁹. Error bar represent standard division. n.d., not detected.

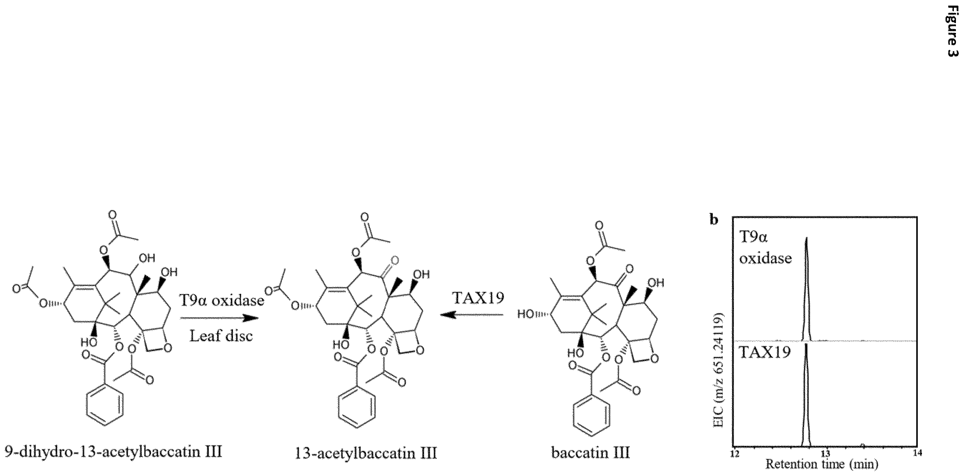

**Fig. 3****. Identification of T9α oxidase.** The 13-acetylbaccatin III could be generated by either T9a oxidase or Tax19. TAX19 is the taxoid-O-acetyltransferases at C13 position^{27,30}.

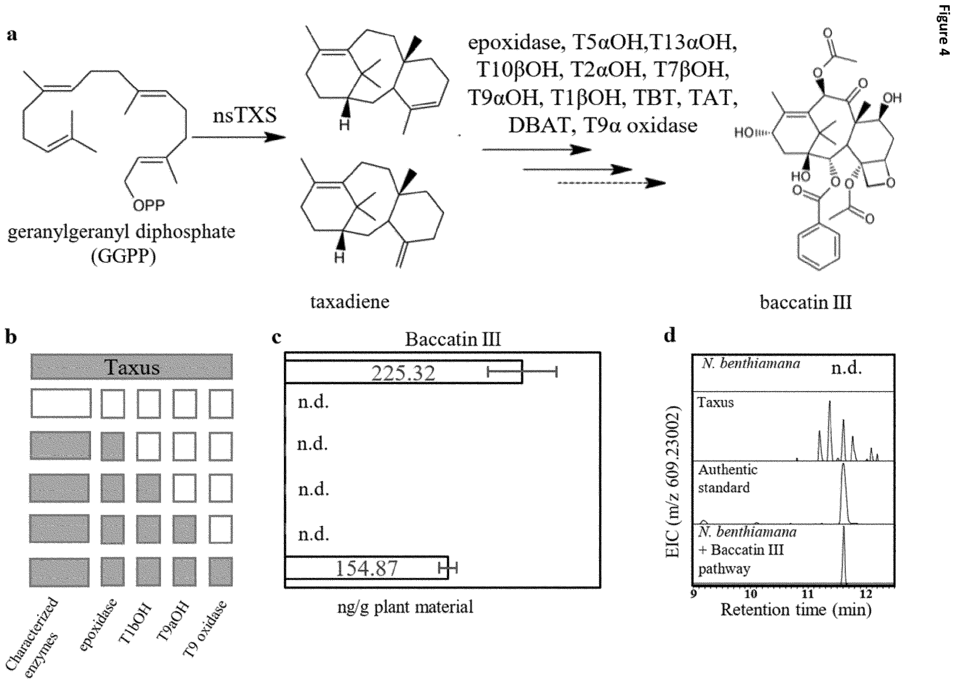

**Fig. 4****. Biosynthesis of Baccatin III in *N. benthamiana.*** a, scheme of Baccatin III biosynthesis. b, expressed the characterized enzymes with new identified four enzymes to produce Baccatin III. c, around 154.87 ng/g of Baccatin III was produced from four biological replicates compared with 225.32 ng/g in Taxus needles. d, the identification of Baccatin III from taxus, authentic standard and infiltrated *N. benthamiana.* Error bar represent standard division. n.d., not detected.

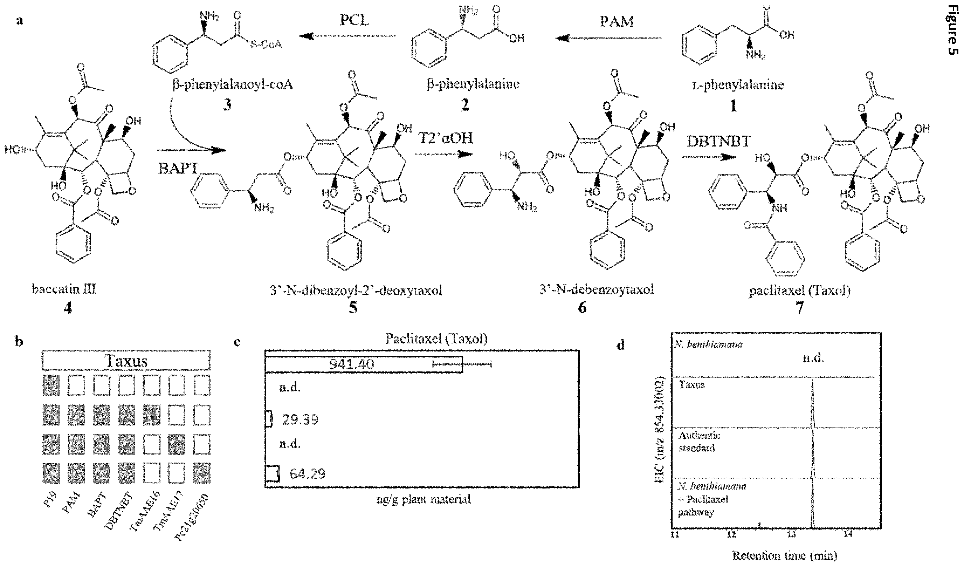

**Fig. 5****. Identification of the** missing **PCL and confirmation of the activity of T2'αOH.** a, scheme of paclitaxel biosynthesis from Baccatin III and L-phenylalanine. b, expressed the characterized three enzymes (PAM, BAPT and DBTNBT) with new identified enzymes to produce the paclitaxel by feeding Baccatin III and L-phenylalanine. c, Paclitaxel was produced by TmAAE16 (~29.39 ng/g) and Pc21g20650 (~64.29 ng/g) in *N. benthamiana,* while taxus needles produced around 941.40 ng/g. d, the identification of Paclitaxel from taxus, authentic standard and infiltrated *N. benthamiana.* Error bar represent standard division. n.d., not detected.

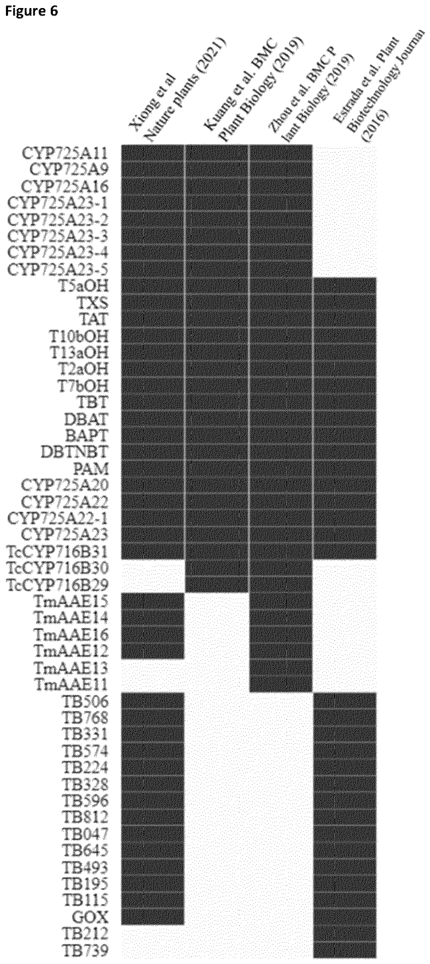

**Fig. 6****. Heatmap of the four published Taxus plants RNA-seq datasets:** Both the MEJA inducing and genome analysis data were obtained from Xiong et al.²⁰. The other RNAseq data was obtained from Me-JA induced Taxus cell lines¹⁶, three tissues²² and three species²³. Three CYP716s were also detected at ten-year-old culture compared with new culture²⁴. Red represent detected as the potential target for paclitaxel biosynthesis.

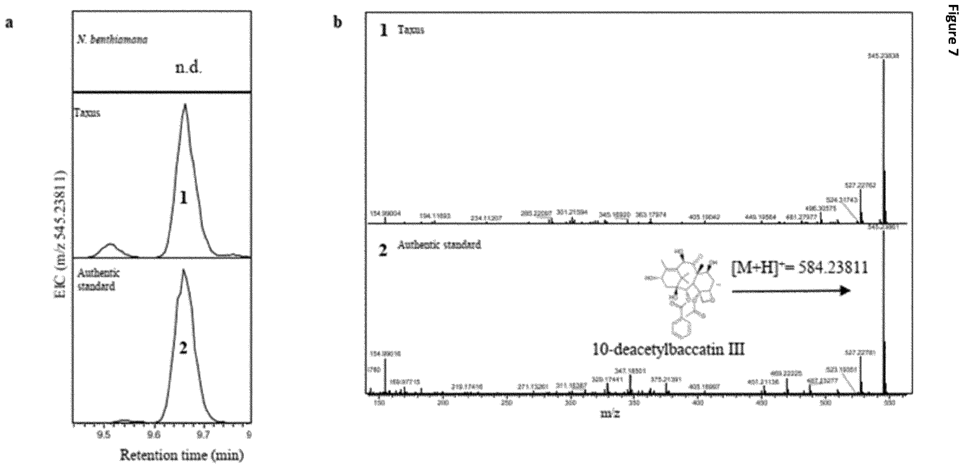

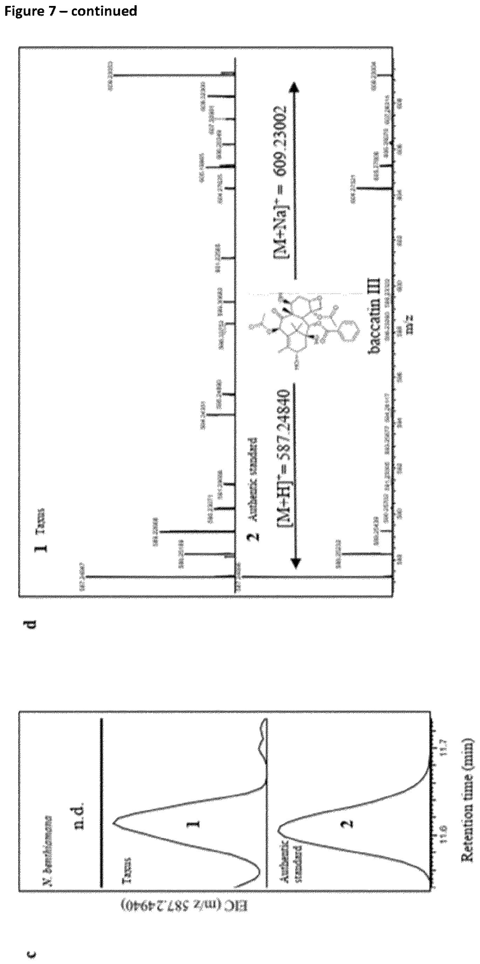

**Fig. 7****. Metabolite analysis of *N. benthamiana, Taxus* and authentic standards by Ultra-high-performance liquid chromatography high-resolution mass spectrometry (UHPLC-HRMS).** *a*, 10-Deacetylbaccatin III identification under positive mode at retention time 9.66. b, MS/MS fragmentation [M+H]⁺= 545.23811 Da, retention time 9.66. c, Baccatin III identification under positive mode at retention time 11.60. d, MS/MS fraction [M+H]⁺= 587.24840 Da and [M+Na]⁺ = 609.23002 Da, retention time11.60. *N. benthamiana* transformed with GFP used as negative control. n.d., not detected.

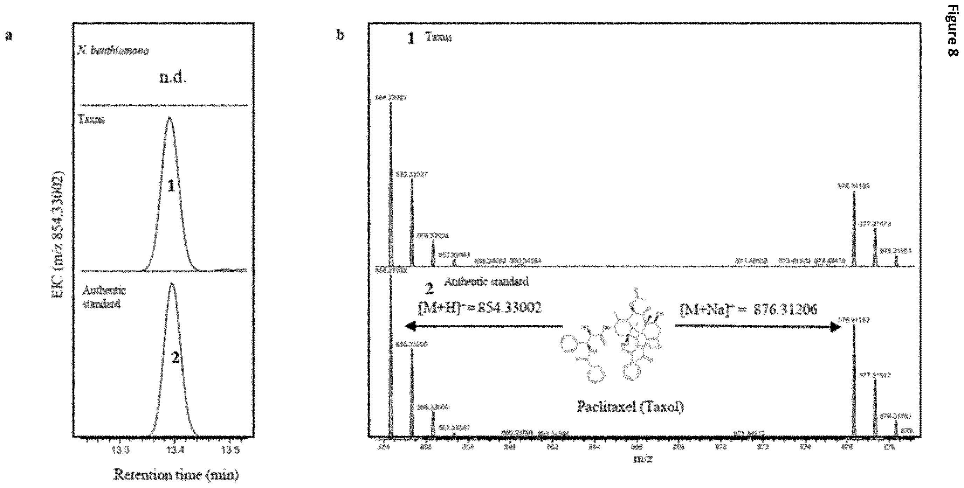

**Fig. 8****. Metabolite analysis of *N. benthamiana, Taxus* and authentic standards by Ultra-high-performance liquid chromatography high-resolution mass spectrometry (UHPLC-HRMS).** a, paclitaxel identification under positive mode at retention time 13.39. b, MS/MS fragmentation [M+H]⁺= 854.33002 Da and MS/MS fragmentation [M+Na]⁺ = 876.32016 Da, retention time 13.39. *N*. *benthamiana* transformed with GFP used as negative control. n.d., not detected.

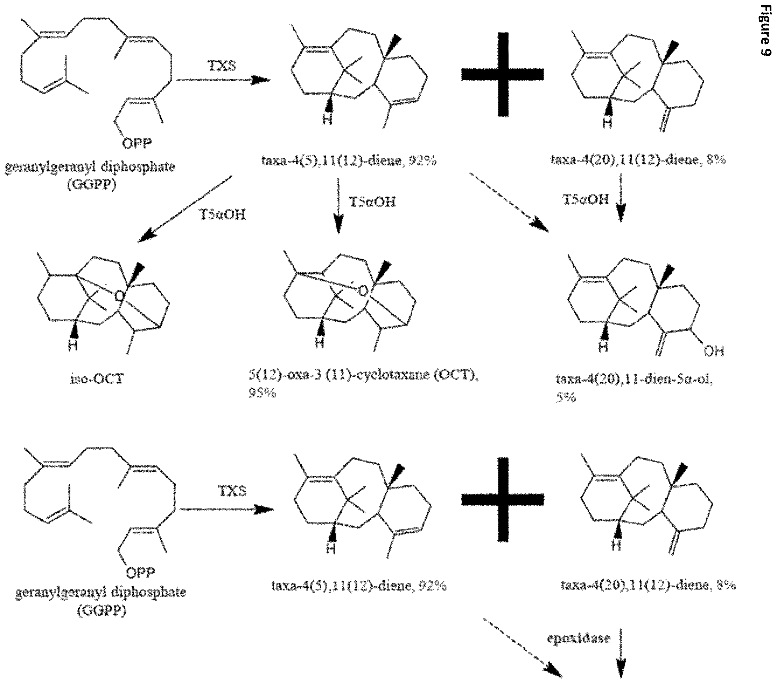

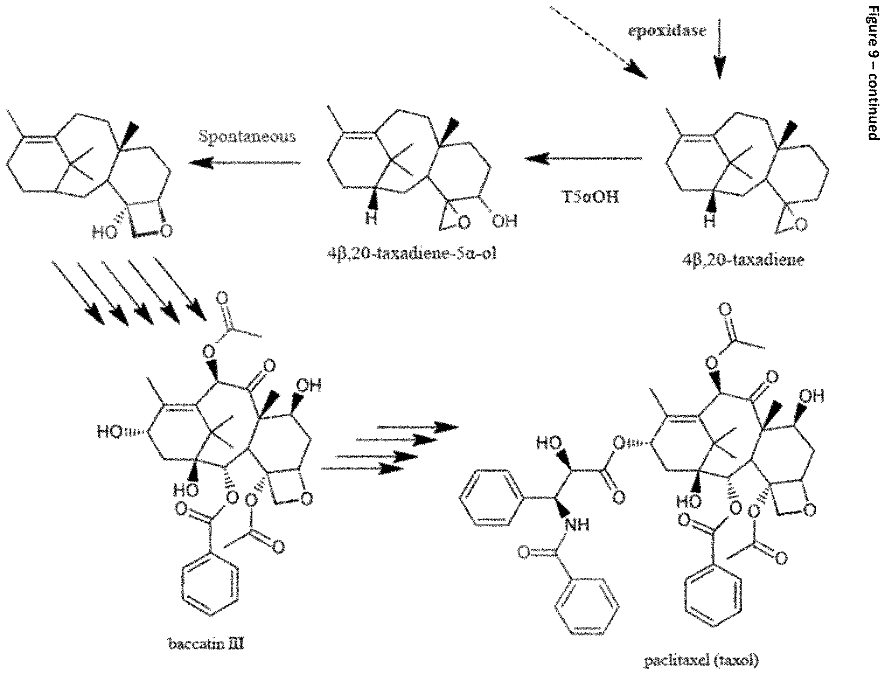

**Fig. 9****. Early Steps of Paclitaxel Biosynthesis** a, The known native pathway catalyzed by TXS and TSaOH to produce OCT with very low amount of taxadiene-5α-ol (~5%). b, the novel C4β,C20-epoxidase could produce 4β,20-taxadienewhich directly feeds the paclitaxel biosynthesis pathway.

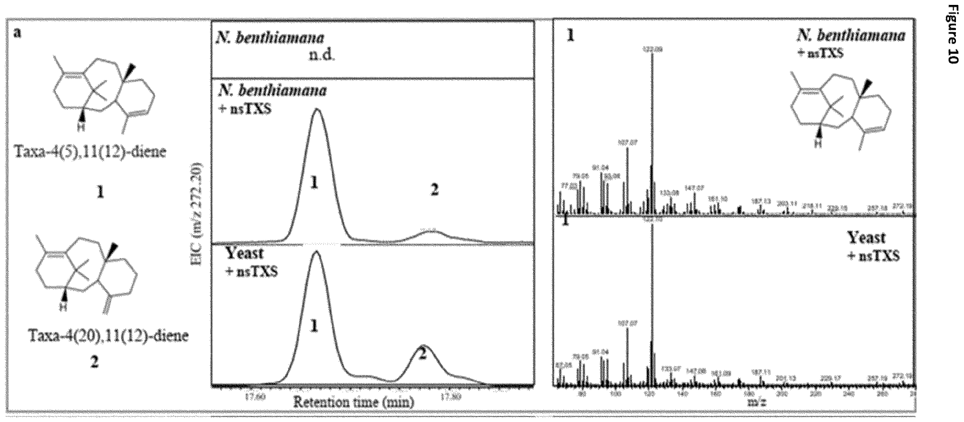

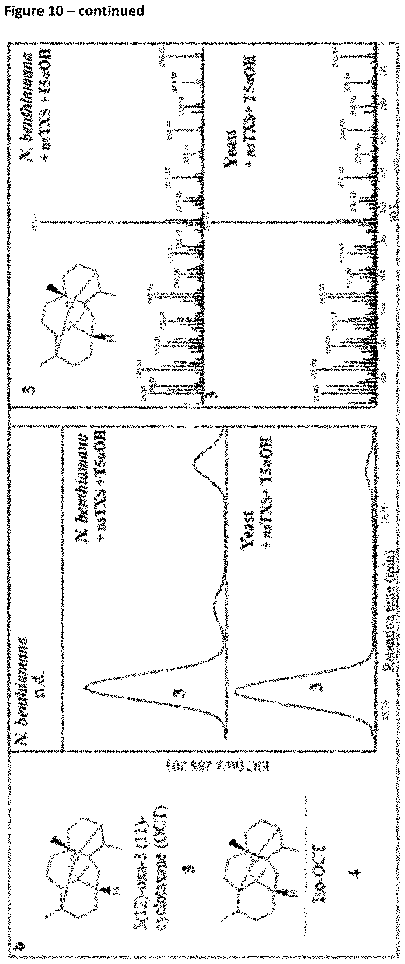

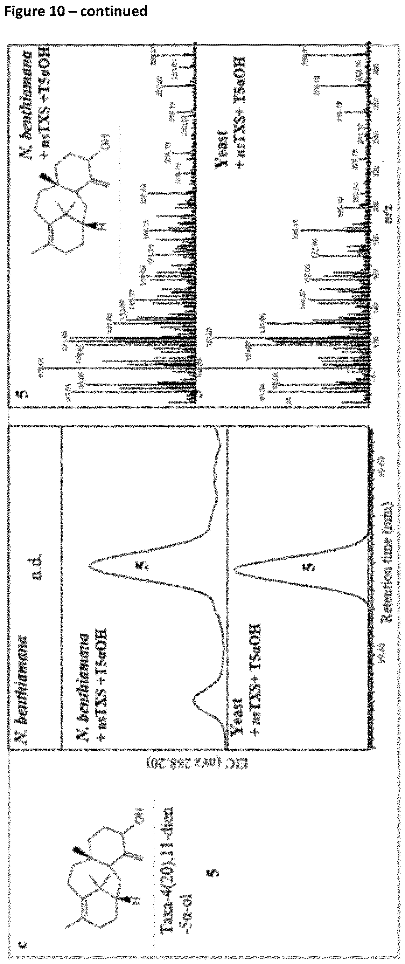

**Fig. 10****. Metabolite analysis of *N. benthamiana* by gas chromatography-mass spectrometry (GC-MS). T**axadiene (a, m/z 272.20), OCT and iso-OCT (b, m/z 288.20) and taxa-4(20),11-dien-5α-ol (c, m/z 288.20) were identified by GC-MS using published Yeast extraction as standard⁴². The truncated TXS (nsTXS) was transiently expressed in *N. benthamiana* with (b, c) or without (a) T5αOH, while the iso-OCT was not characterized in the yeast extract by NMR. *N. benthamiana* transformed with GFP used as negative control. n.d., not detected.

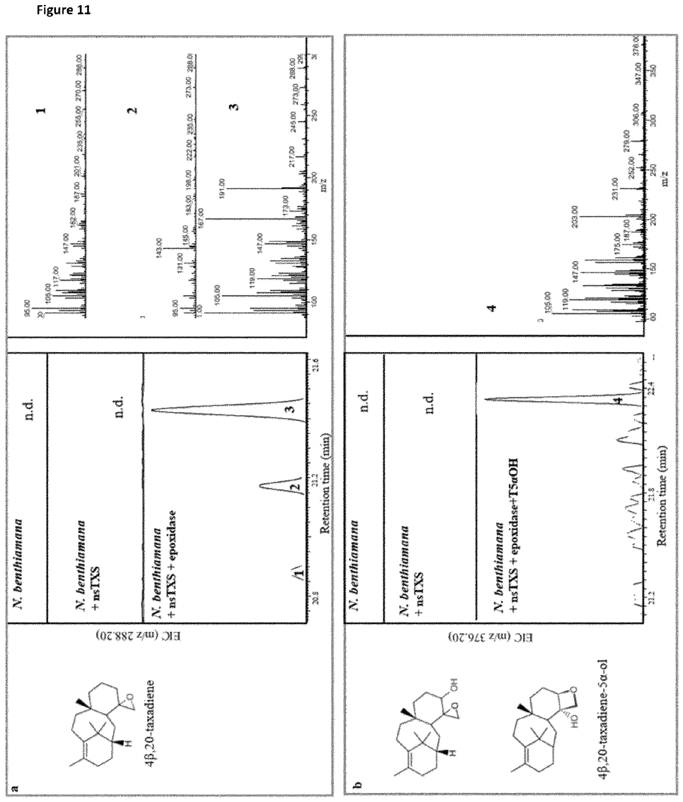

**Fig. 11****. Metabolite analysis of *N. benthamiana* by N,O-bis(trimethylsilyl)trifluoroacetamide (BSTFA) derivatized gas chromatography-mass spectrometry (GC-MS).** 4β,20-taxadiene (a, m/z 288.20) and 4β,20-taxadiene-5α-ol (b, m/z 376.20 with 1 TMS) were identified by GC-MS. 4β,20-taxadiene could not be derivatized by BSTFA indicating the oxygen ring formation. *N. benthamiana* transformed with GFP used as negative control. n.d., not detected.

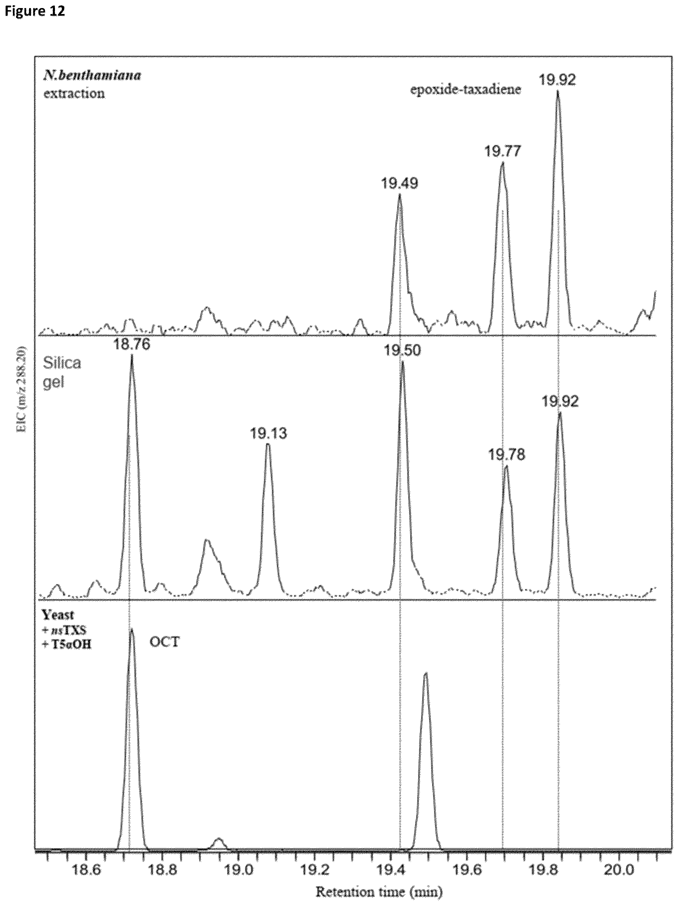

**Fig. 12****. Instability of 4β,20-taxadiene in silica-gel column.** The transiently overexpressed nsTXS and epoxidase *N. benthamiana* was extracted by ethyl acetate. The extraction was significantly degraded in silica gel column, Al₂O₃ column and C18 column at HPLC.

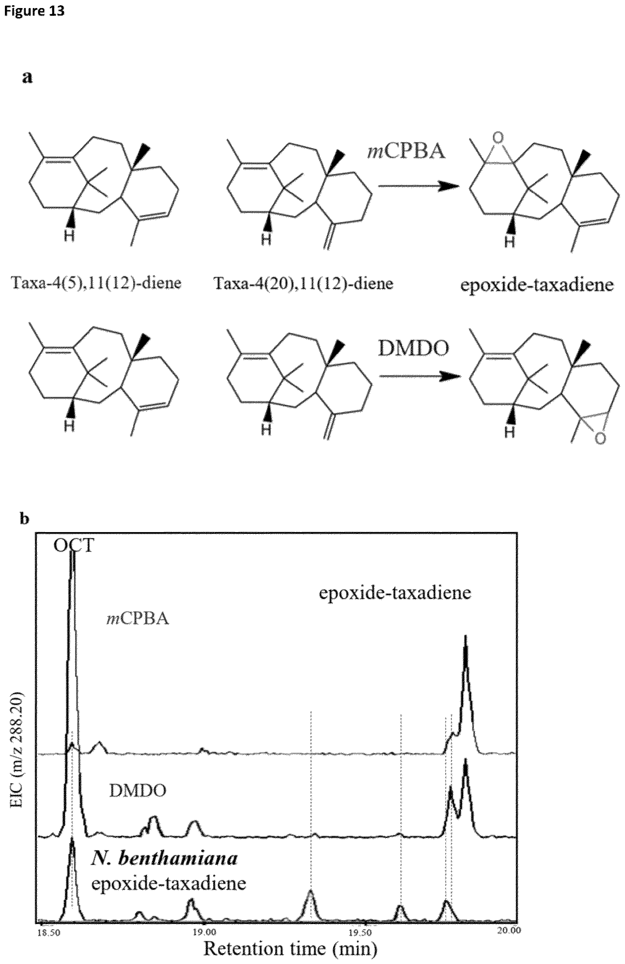

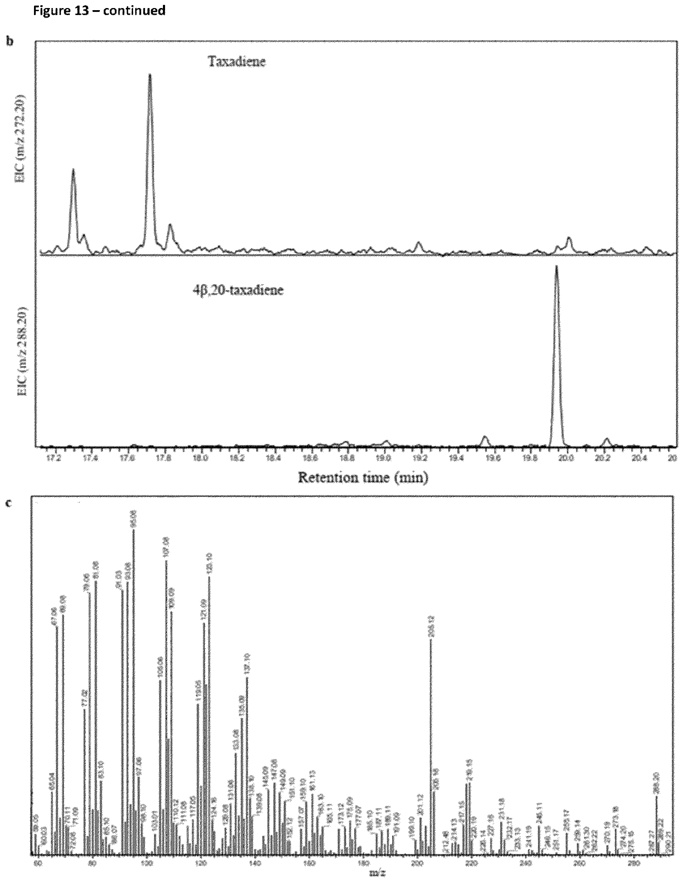

**Fig. 13****. Chemical treatment of taxadiene.** Both mCPBA and DMDO (a) were used to produce the epoxide-taxadiene²⁶. b, there are no overlap between the *N. benthamiana* produced epoxide-taxadiene and chemical synthesized epoxide-taxadiene. c, the mass profile at retention time 19.97 is not similar between *N. benthamiana* produced epoxide-taxadiene and chemical synthesized epoxide-taxadiene.

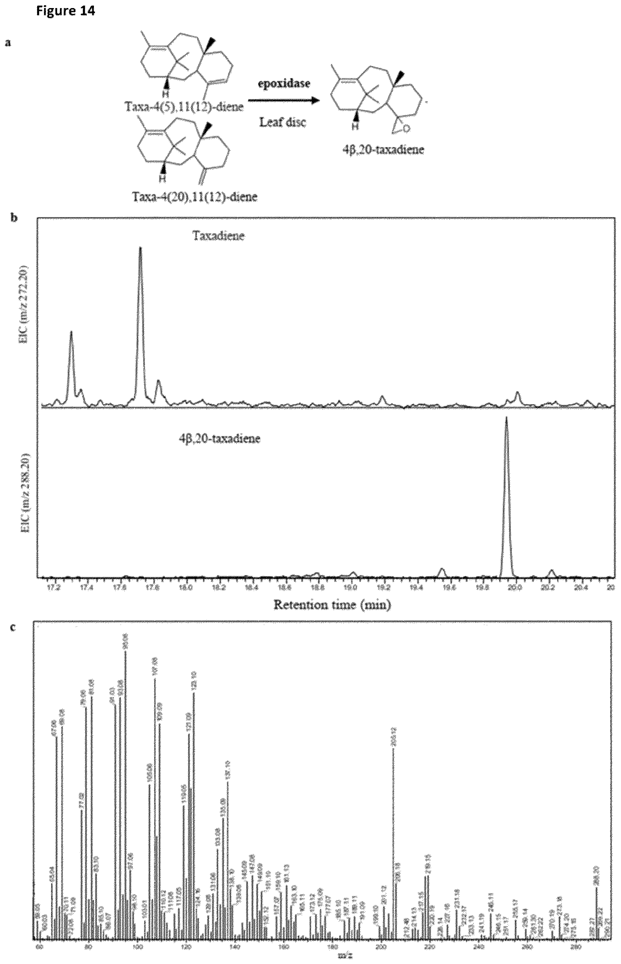

**Fig. 14****. Enzyme assay of taxadiene by leaf disc expressed with epoxidase.** a, scheme of epoxidase enzyme assay. b, the taxadiene and 4β,20-taxadiene were detected by GC-MS. c, mass profile of 4β,20-taxadiene.

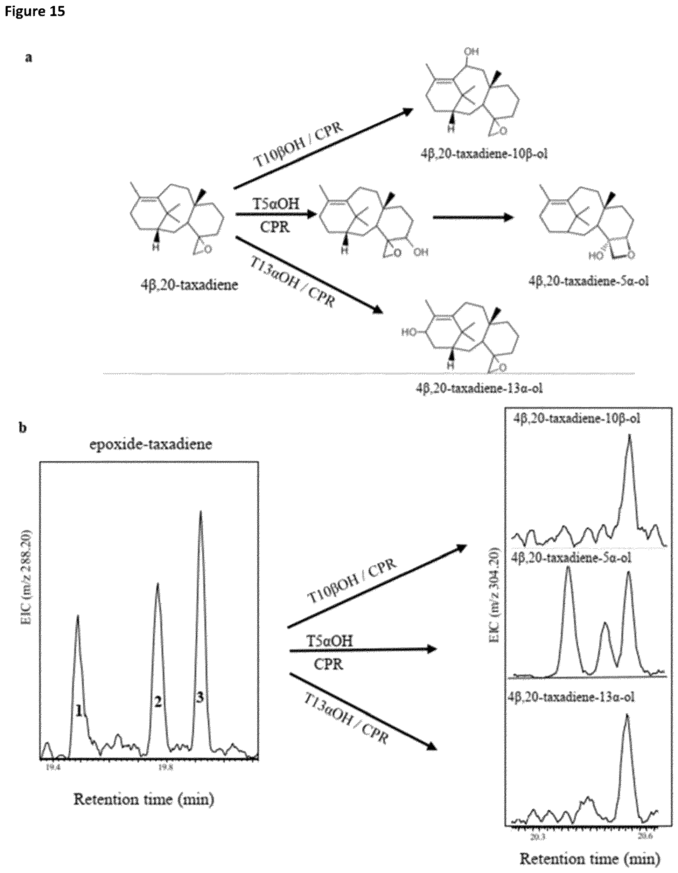

**Fig. 15****. Enzyme assay of 4β,20-taxadiene by T5αOH, T13αOH and T10βOH.** a, scheme of enzyme assay. The ethyl acetate extracted epoxide-taxadiene were treated byTSaOH, T13αOH or T10βOH with Cytochrome P450 reductase. b, both peak 2 and peak 3 could be recognized by three hydroxylases to produce the potential products.

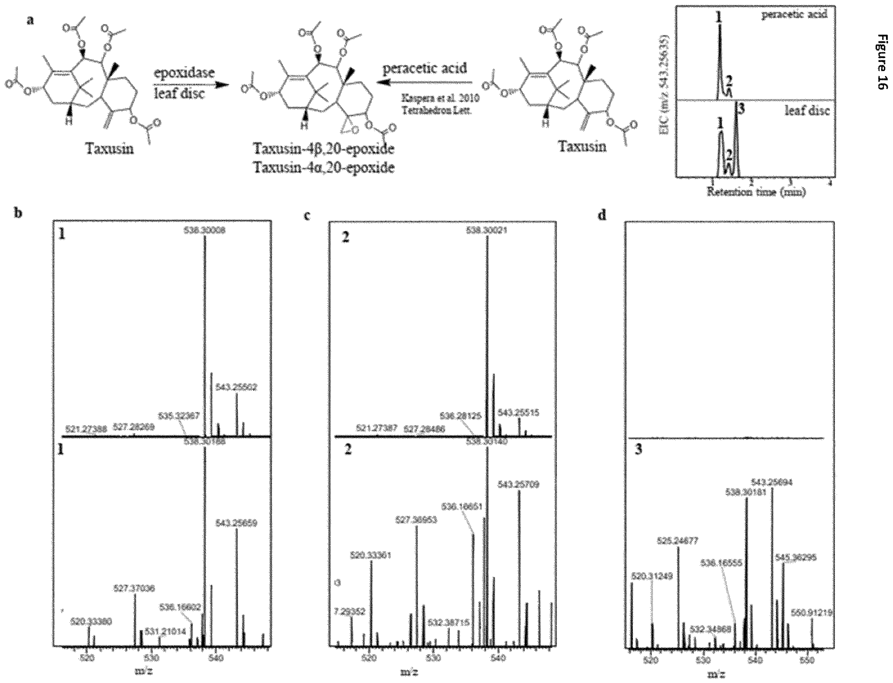

**Fig. 16****. Enzyme assay of epoxidase.** a, scheme of taxusin-4β,20-epoxide and taxusin-4α,20-epoxide produced by both enzyme assay and chemical synthesis (peracetic acid)²⁹. The mass profiling of three peaks (b, c, d) were identified by UPLC-MS/MS. Both peak 1 and 2 are overlapped at retention time and mass profiling, while the peak 3 are unique peak from enzyme assay. Taxusin-4β,20-epoxide and Taxusin-4α,20-epoxide were detected as MS/MS fraction [M+Na]⁺ = 543.25635 Da.

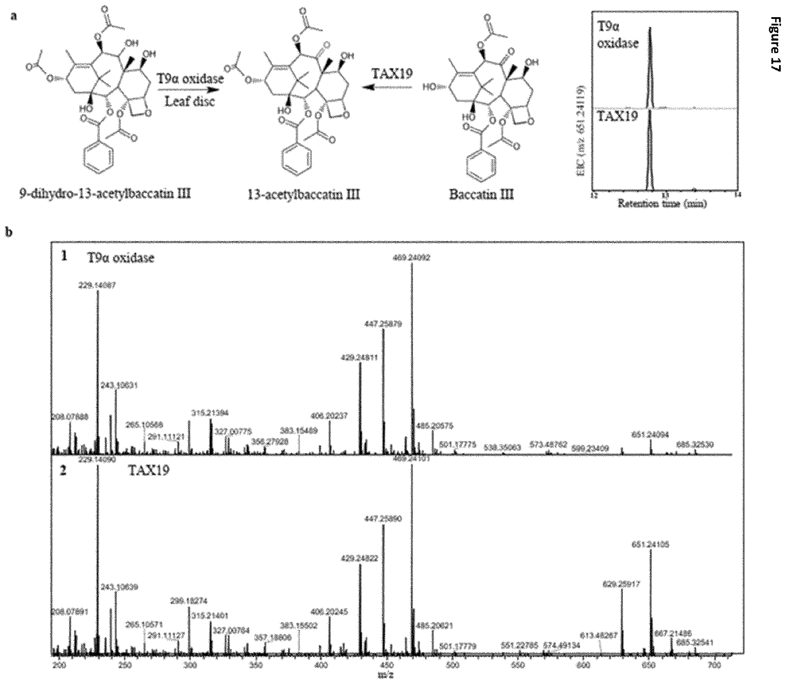

**Fig. 17****. Identification of T9α oxidase.** a, scheme of the 13-acetylbaccatin III biosynthesis from either T9a oxidase or Tax19. b, mass profile of 13-acetylbaccatin III from two substrates and enzymes. 13-acetylbaccatin III was detected as MS/MS fraction [M+Na]⁺ = 651.24119 Da, retention time12.76. TAX19 is the taxoid-O-acetyltransferases at C13 position^{27,30}.

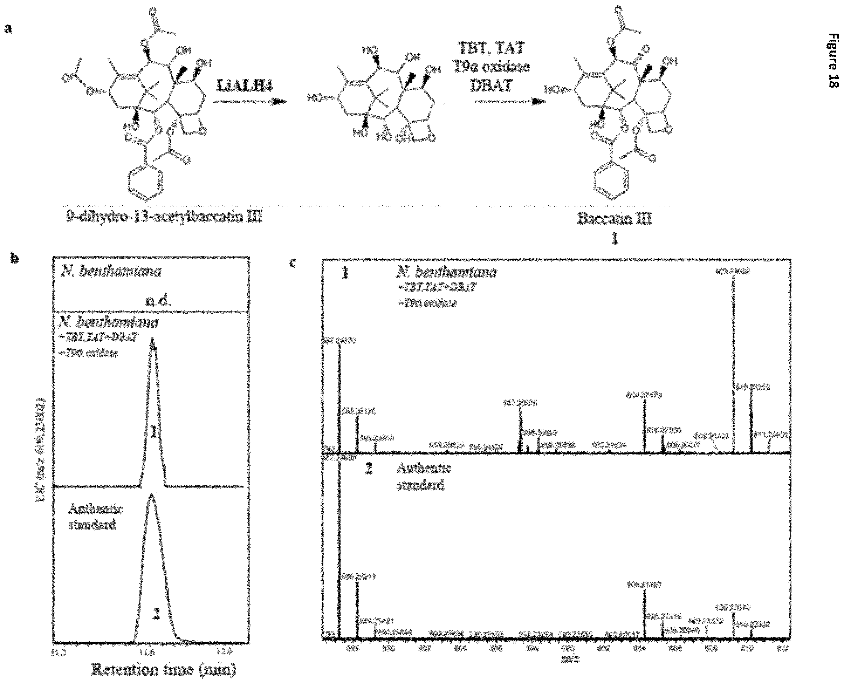

**Fig. 18****. Confirmation the function of T9oxidase in baccatin III biosynthesis.** a, 9-dihydro-13-acetylbaccatin III was deacetylated by LiALH4 (same reaction as taxusin) and feed to *N. benthamiana with* TBT, TAT, DBAT and T9 α oxidase. b, the baccatin was generated by feeding deacetyl-9-dihydro-13-acetylbaccatin III. Baccatin III was detected as MS/MS fraction [M+H]⁺= 587.24840 Da and [M+Na]⁺ = 609.23002 Da, retention time 11.60. *N. benthamiana* transformed with GFP used as negative control. n.d., not detected.

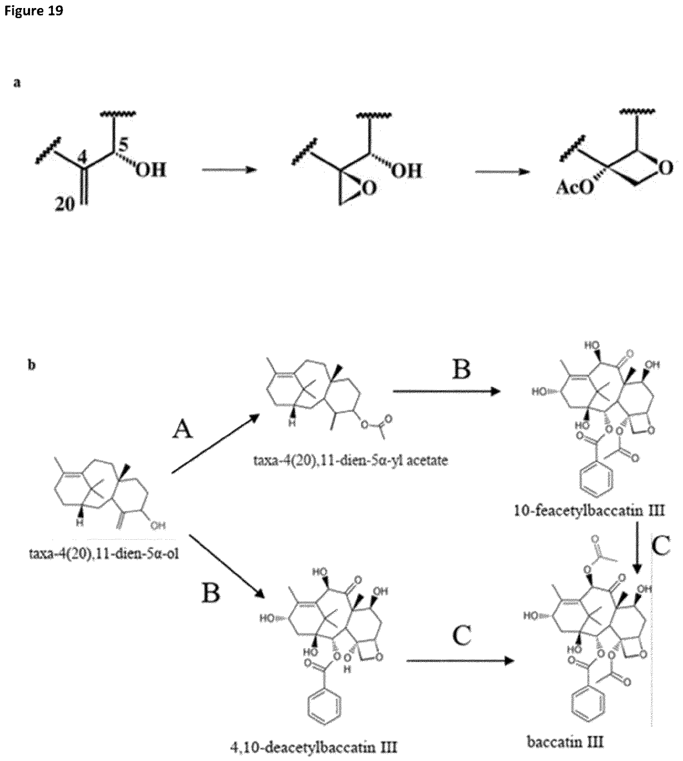

**Fig. 19****. Oxetane ring formation in former publications. a,** Proposed conversion of taxa-4(5),11(12)-diene to taxa-4(20),11(12)-dien-5-ol, and transformation of the 4(20)-ene5-oxy functional grouping to the corresponding oxirane and oxetane groups⁴³. b, proposed biosynthetic matrix to Baccatin III, showing parallel routes with alternative acetylation sequences. A: 5-O-Acetylation by taxadiene acetyl transferase. B: Several oxidation and acylation reactions. C: 10-Deacetylbaccatin III acetyltransferase-catalyzed acetylation ^{14,27}.

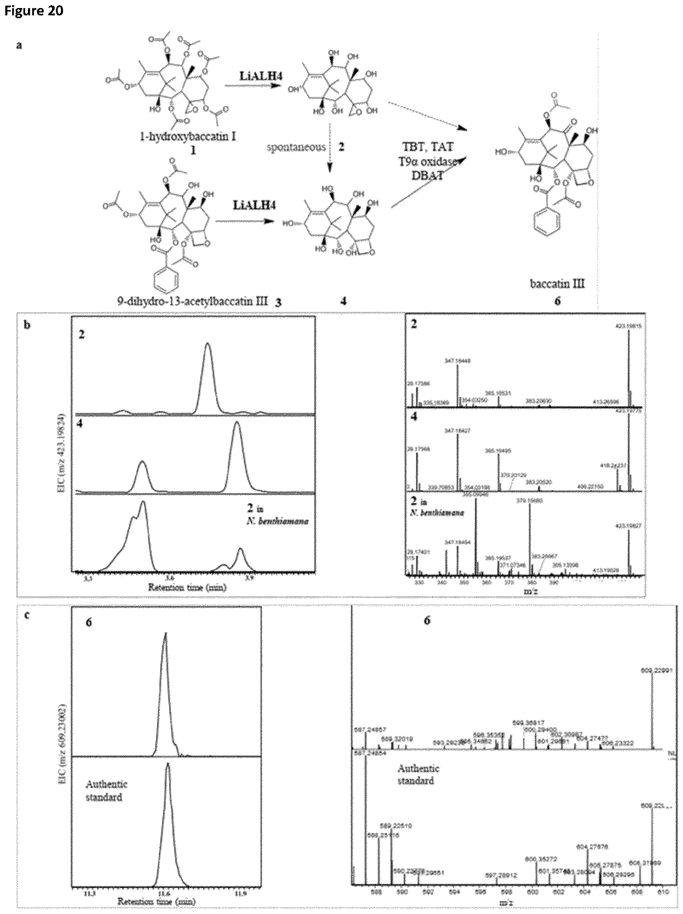

**Fig. 20****. Oxetane ring formation in synthetic chemical.** a, scheme of deacetyl-1-hydroxybaccatin I (2) oxetane ring formation. Both 1-hydroxybaccatin I and 9-dihydro-13-acetylbaccatin III were deacetylated by LiALH4 same as reaction of taxusin¹⁴. b, the deacetyl-1-hydroxybaccatin I (2) could be transferred to deacetyl-9-dihydro-13-acetylbaccatin III (4) in *N. benthiamana.* c, the production of baccatin III from deacetyl-1-hydroxybaccatin I in *N. benthiamana.* Four enzymes (TBT, TAT, T9a oxidase, DBAT) were co-expressed in *N. benthiamana* leaves. Baccatin III was produced by feeding deacetyl-1-hydroxybaccatin I. Baccatin III was detected as MS/MS fraction [M+H]⁺= 587.24940 Da and [M+Na]⁺ = 609.23302 Da, retention time 11.60.

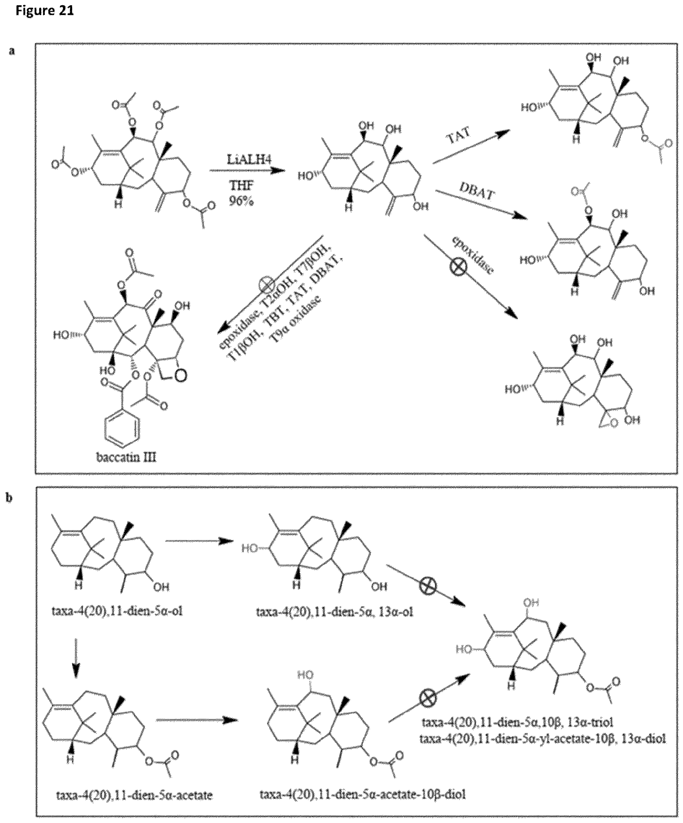

**Fig. 21****. Feeding experiment of deacetylated Taxusin.** a, scheme of feeding experiment. Only the acetylated chemicals could be detected. The C5 hydrolyzed product could not be catalyzed by epoxidase. When feed to transient expressed eight genes (epoxidase, T2αOH, T7βOH, T1βOH, TBT, TAT, DBAT, T9α oxidase) *N. benthiamana,* no baccatin III or similar mass products were produced. b, Co-expressed TAT, T5αOH, T10βOH and T13αOH could not produce Taxa-4(20),11-dien-5α,10β, 13α-triol or Taxa-4(20),11-dien-5α-yl-acetate-10β, 13α-diol in previous publication^{14,44}. Red sign represents impossible pathway.

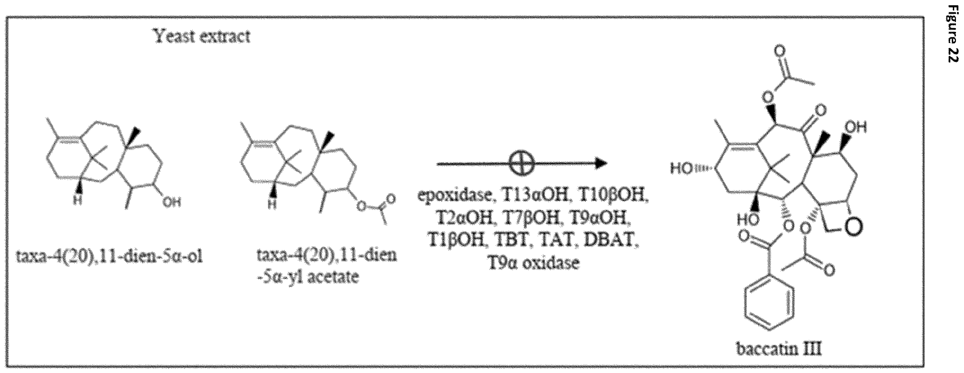

**Fig. 22****. Feeding experiment of yeast,** the yeast extracted taxa-4(20),11-dien-5α-ol and taxa-4(20),11-dien-5α-yl acetate⁴² were feed into *N. Benthiamana* with transiently expressed eleven genes, while neither baccatin III nor a chemical of similar mass were produced. Red sign represents impossible pathway.

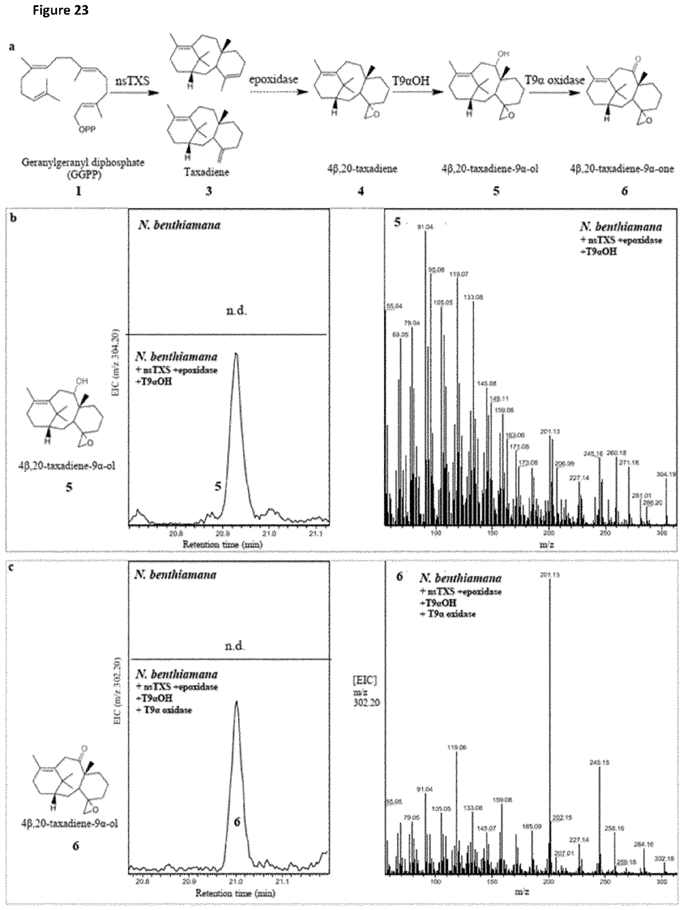

**Fig. 23****. Identification of T9αOH.** a, scheme of generate the 4β,20-taxadiene-9α-ol (5) and 4β,20-taxadiene-9α-one (6). b, truncated TXS was co-expressed with both epoxidase and T9αOH to generate the potential 4β,20-taxadiene-9α-ol (5) which was identified by GC-MS. c, the 4β,20-taxadiene-9α-ol (5) could be catalyzed by T9α oxidase as 4β,20-taxadiene-9α-one (6). This candidate was later confirmed by Baccatin III biosynthesis. *N. benthamiana* transformed with GFP used as negative control. n.d., not detected.

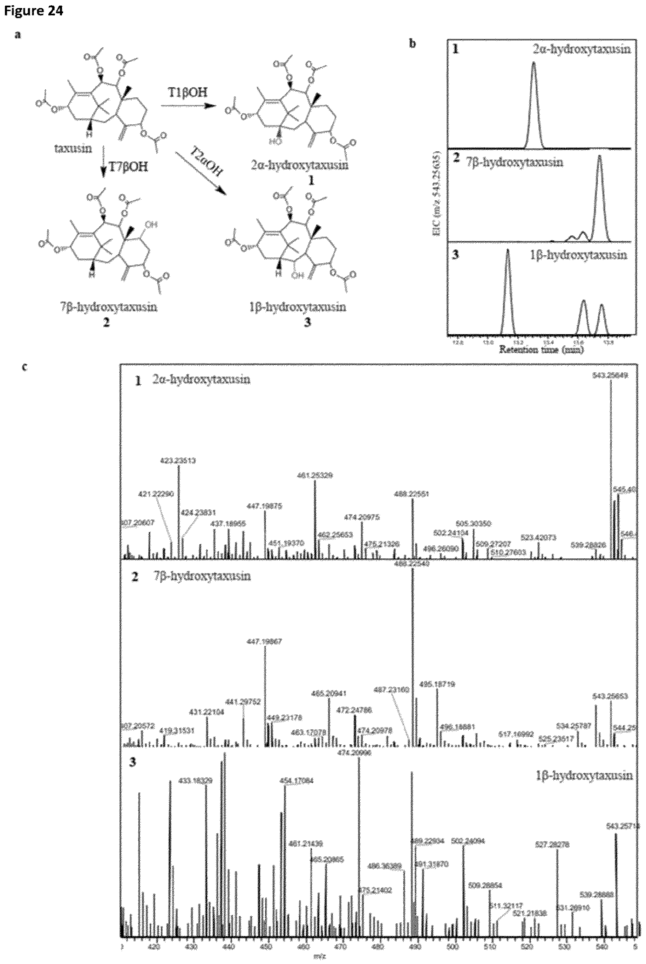

**Fig. 24****. Identification of T1βOH.** a, scheme of generate the 2α-hydroxytaxusin (1)⁴⁵, 7β-hydroxytaxusin (2)³² and 1β-hydroxytaxusin (3). b, three chemicals are separately generated by feeding Taxus in *N*. *benthamiana* with transiently expressed each gene. c, mass profiling of three peaks with different retention time in UPLC-MS.

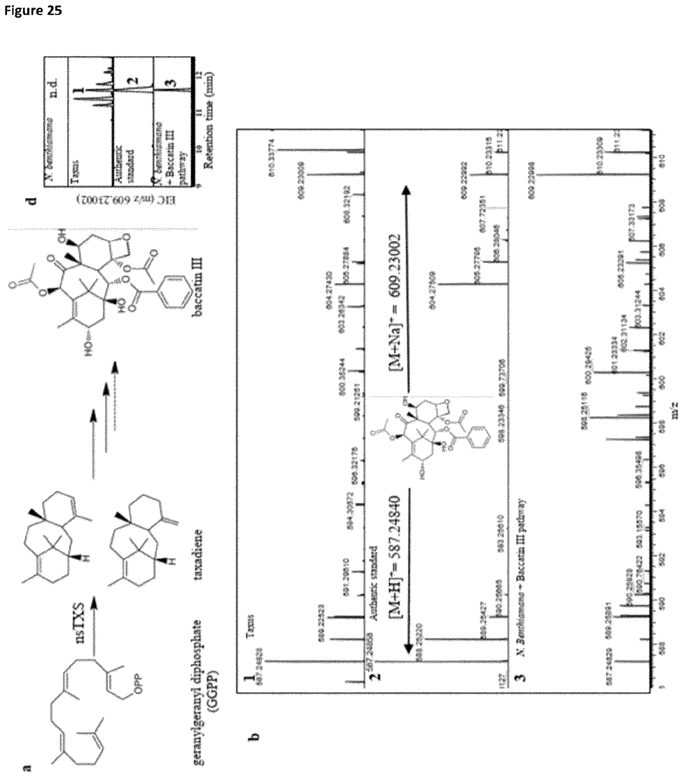

**Fig. 25****. Identification of baccatin III in *N. benthamiana.*** a, scheme of baccatin III biosynthesis. b, mass profiling of three peaks of baccatin III. As baccatin III was not generated in *N. benthamiana,* baccatin III was detected as MS/MS fraction [M+H]⁺= 587.24840 Da and [M+Na]⁺ = 609.23002 Da, retention time11.60. *N. benthamiana* transformed with GFP used as negative control. n.d., not detected.

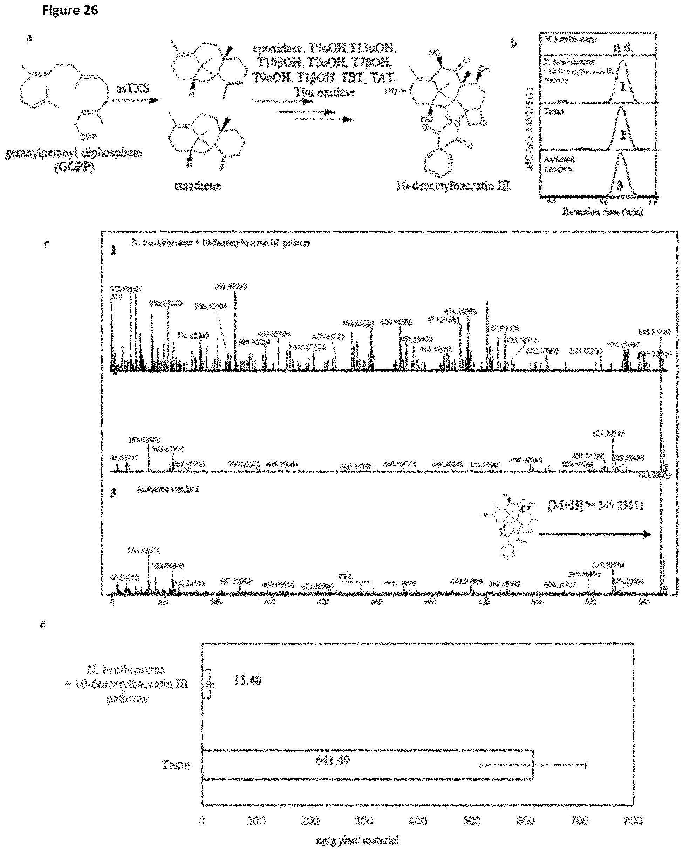

**Fig. 26****. Biosynthesis of 10-deacetylbaccatin III in *N. benthamiana.*** a, scheme of 10-deacetylbaccatin III biosynthesis. b, expressed the characterized enzymes with new identified four enzymes to produce the 10-deacetylbaccatin III. c, mass profiling of three peaks of 10-deacetylbaccatin Ill. As 10-deacetylbaccatin III was not generated in *N. benthamiana,* 10-deacetylbaccatin III was detected as MS/MS fraction [M+H]⁺= 545.23811 Da, retention time 9.68. c, around 154.07 ng/g of 10-deacetylbaccatin III was produced from four biological replicates compared with 6414.58 ng/g in Taxus needles. d, the identification of baccatin III from taxus, authentic standard and transiently expressed *N. benthamiana. N. benthamiana* transformed with GFP used as negative control. Error bar represent standard division. n.d., not detected.

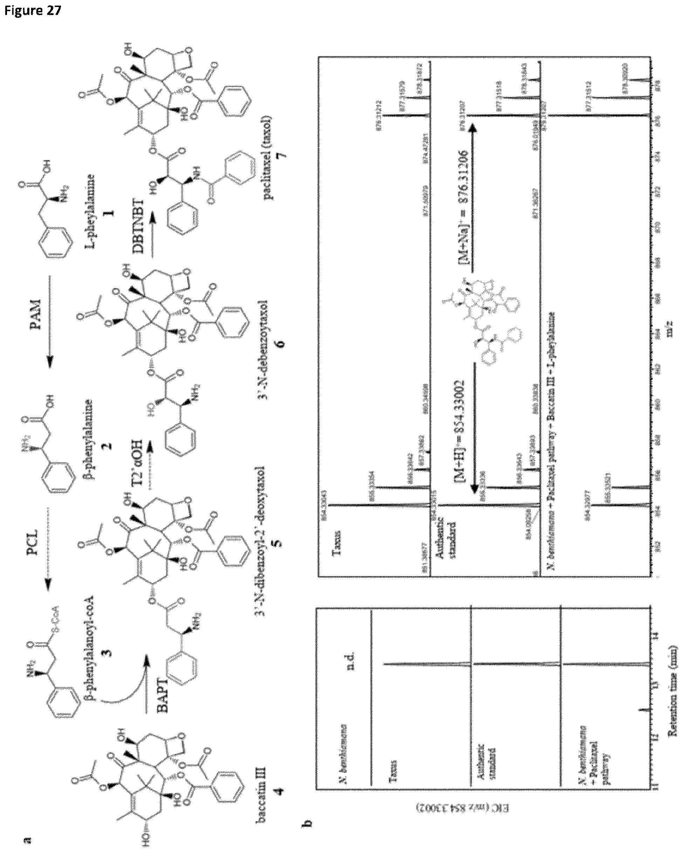

**Fig. 27****. Identification of Paclitaxel in *N. benthamiana.*** a, scheme of paclitaxel biosynthesis from baccatin III and L-phenylalanine. b, mass profiling of three peaks of Paclitaxel. As Paclitaxel was not generated in *N. benthamiana,* the paclitaxel was identified by its MS/MS fragmentation [M+H]⁺ = 854.33002Da, RT 13.40 and MS/MS fragmentation [M+Na]⁺ = 876.31207Da, RT 13.40. n.d., not detected.

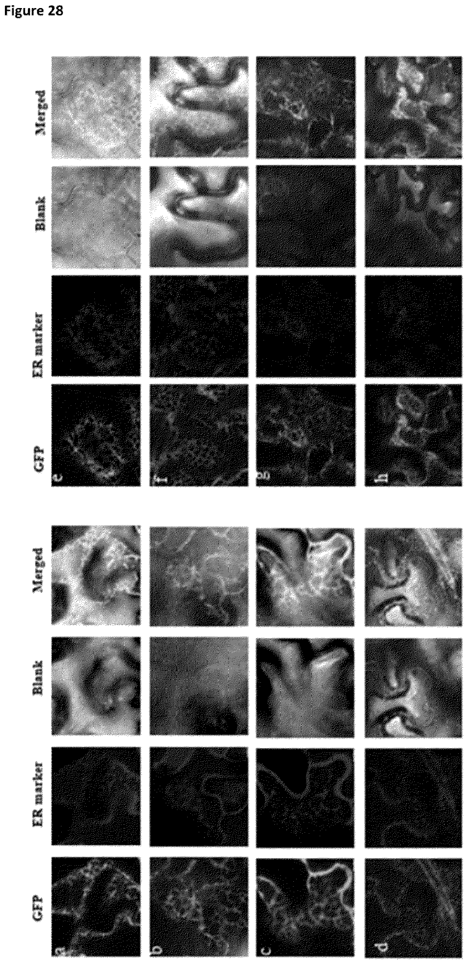

**Fig. 28****. Sub-cellular-localization of newly identified enzymes.** Enzymes with C-terminal GFP and 35s promoter were transiently expressed in *N. benthamiana.* The ER sub-cellular-localized epoxidase (a), T5aOH (b), TAT (c), CYP725A22-1 (d), CYP725A23-1 (e), CYP725A31(f), T2'αOH (g) and PCL (h) with ER mCherry marker were detected by confocal microscopy.

The examples illustrate the invention.

### Example 1- Introduction

As a major source of bioactive small molecules, medicinal plants represent important resources for treatments against several human diseases. Since the mid-1800s, the toxicity of Taxaceae extracts to animals attracted the attention of chemists¹. An unusual cyclic diterpenoid (paclitaxel) was isolated and characterized from the bark of the Pacific yew (*Taxus brevifolia*)², and was later shown to bind to β-tubulin, stabilizing the assembly of microtubules. This activity of paclitaxel results in the slowing or blocking of mitosis at the metaphase-anaphase transition, and ultimately causes the induction of apoptotic cell death³. Since 1992, paclitaxel, under the brand name Taxol, was approved by the Food and Drug Administration (FDA) for medical use against several solid cancers (ovarian, breast, lung, cervical, pancreatic, and Kaposi's sarcoma)⁴. Since then, Taxol, and some of its related taxane analogs (e.g., docetaxel), have become leading anti-cancer drugs with sales reported to exceed five billion per year⁵. Originally, paclitaxel was directly extracted from twigs, bark, or needles of pacific yew, but the low yield and the amount generally required for a therapeutic cycle (2-3 g paclitaxel/patient) have intensified the search for alternative approaches to produce paclitaxel⁶. Total chemical synthesis of paclitaxel, achieved for the first time in 1994, is not an economically sustainable alternative⁷. Paclitaxel production for medical use is currently based on a semi-synthetic approach, using 10-deacetylbaccatin III isolated from *Taxus* needles as a starting material for chemical synthesis. Plant cell cultures derived from several *Taxus* species are also employed for commercial paclitaxel production. Ideally, metabolic engineering approaches could be used to enhance paclitaxel levels in *Taxus* cell culture⁸⁻¹⁰ or to reconstitute paclitaxel production in in a heterologous host such as *Saccharomyces cerevisiae¹¹.* However, the biosynthetic pathway for paclitaxel must first be fully elucidated for metabolic engineering strategies to be feasible.

The biosynthesis of taxadiene (taxa-4(5),11(12)-diene), a C₂₀ terpenoid, is the first committed step in the production of paclitaxel. Taxadiene is formed in the plastid by the action of taxadiene synthase (TXS)¹², an enzyme using as substrate the geranylgeranyl pyrophosphate (GGPP) produced through the methylerythritol phosphate pathway (MEP)¹³. Taxadiene is then presumably transported to the endoplasmic reticulum (ER), where it is further modified by at least 19 enzymes¹⁴. In particular, taxadiene is hydroxylated at C1, C2, C5, C7, C9, C10, and C13 with further oxidation occurring at C9^{14,15} (Fig. 1). After further modifications, including acetylation and formation of the oxetane ring, the resulting intermediate (baccatin III) is the target of the final decoration steps, which include the attachment of a β-phenylalanoyl moiety on C-13, additional oxidation on C-2α and the transfer of a benzoyl moiety on the same β-phenylalanoyl side chain (Fig. 1). The enzymes responsible for twelve of these steps have been identified and well characterized^{16,17,18}, but for at least six additional hypothetical reactions, presumably catalyzed by phenylalanine-CoA ligase (PCL), taxane 1β-hydroxylase (T1βOH), taxane 9α-hydroxylase (T9αOH), and taxane 9α-dioxygenase (T9oxidase) molecular evidence is lacking, despite numerous omics resources that are now available for *Taxus,* including the recent release of the genome sequence of *Taxus chinensis var. mairei*^{15,19,20}. The following examples the described the identification of these missing paclitaxel biosynthetic enzymes, verified by the reconstitution of baccatin III and paclitaxel in the heterologous host *N. benthamiana* (Fig. 1).

### Example 2 - Identification of candidate genes completing the pathway of paclitaxel biosynthesis

Genes of plant specialized biosynthetic pathways are often co-expressed²¹. This characteristic was utilized to search for candidate genes for the missing steps of paclitaxel biosynthesis across multiple published RNAseq datasets using the twelve previously characterized paclitaxel biosynthesis genes as bait^{16,22-24}(Fig. 6). From these RNAseq datasets, expression of the 13 characterized paclitaxel biosynthesis genes could be detected, while a further 37 uncharacterized potential biosynthetic gene candidates were found to be co-expressed with these identified genes (Table 1). Given the high complexity of paclitaxel biosynthesis, the pathway was split into two modules; (i) 10-deacetylbaccatin III and baccatin III biosynthesis and (ii) the late stages of paclitaxel production from baccatin III (Fig. 1).

### Example 3 - Early oxidation reactions on taxadiene

The biosynthetic genes for the biosynthetic intermediate taxadiene have been successfully transferred to *E.coli²⁵*, yeast¹¹, and tobacco chloroplasts ^{6,13}. However, how the pathway proceeds immediately downstream is less clear. It is proposed that the CYP725A4 taxane-5α-hydroxylase (TSaOH) converts taxadiene into taxadiene-5α-ol, but enzyme assays show that TSaOH produces primarily 5(12)-oxa-3 (11)-cyclotaxane (OCT), which can no longer re-enter the route to paclitaxel^{6,8,11,26} (Figs. 7-10). An alternative biosynthetic hypothesis was formulated, in which some oxidized form of taxadiene is generated by yet uncharacterized enzyme, and then TSaOH acts on this oxidized form of taxadiene to create the oxetane ring^{14,27} (Fig. 9).

To test this hypothesis, production of taxadiene was firstly optimized in *N. benthiamana* using previously reported approaches. Truncated versions of the diterpene synthase TXS (nsTXS) and geranylgeranyl pyrophosphate synthase (nsGGPS) were transiently expressed that were targeted to the cytosol instead of the plastid, along with the rate-limiting enzyme of the mevalonate pathway, 3-hydroxy-3-methylglutaryl-CoA reductase (HMGR)^{13 6,13,28}. Approximately 100 µg of taxadiene per gram fresh weight of *N. benthiamana* leaf was obtained.

With an optimized taxadiene production platform in hand, screening the biosynthetic gene candidates that encoded oxidases was set about to see if one of these candidates could use taxadiene as a substrate. When one of the candidates, encoding a 2-oxoglutarate (2OG)/Fe(II)-dependent dioxygenase (2ODD, epoxidase)¹⁶, was expressed in *N. benthamina* along with nsTXS1, nsGGPS and HMGR, three ion peaks were observed related to a compound with *m*/*z* 288 following derivatization-based GC-MS analysis of an ethyl acetate extract of the *N. benthiamana* leaf tissue (Fig. 11). None of these spectra matched OCT, iso-OCT, taxadiene-5α-ol or chemically synthesized epoxide-taxadiene (Figs. 12-13). It was attempted to express this 4β-C20 epoxidase in *E. coli* or *S*. *cerevisiae* strains that produced taxadiene. However, in both instances only the taxadiene starting material could be detected, suggesting that this enzyme cannot be expressed in active form in these expression systems. However, when T5αOH (Fig. 2 and Fig. 11), along with HMGR, nsGGPS, nsTXS, epoxidase, was transiently expressed oxidized taxadiene was significantly reduced, suggesting that it is a productive intermediate for the downstream enzymes of the paclitaxel pathway. Although the products produced by epoxidase and T5αOH could not be characterized, the putative 4β,20-taxadiene-5α-ol (6, Fig. 2) had a mass of 376.20 and appeared to consistent with a taxadiene scaffold with C4-20 oxidation (Fig. 2b and Fig. 11).

Moreover, using the synthesized taxusin-4β,20-epoxide and taxusin-4α,20-epoxide as authentic standard²⁹, it was able to demonstrate that the epoxidase could catalyze the taxusin to the taxusin-4β,20-epoxide and taxusin-4α,20-epoxide (HRMS m/z 543.25572 [M⁺+Na]) conversions (Fig. 2c and Fig. 16). This data shows that the epoxidase catalyzes an epoxidation of C4-20, although it was not yet possible to isolate any of the epoxidase products in quantities sufficient for NMR.

### Example 4 - Discovery of the missing T9α oxidase and oxetane ring formation

An oxidase that acts on carbon 9 (T9 **α** oxidase) is anticipated to be essential for the formation of baccatin III, but the responsible protein has not yet been identified. Supplying 9-dihydro-13-acetylbaccatin III as substrate to tobacco leaves expressing one of the gene candidates, which was homologous to gibberellin 20-oxidase, resulted in the production of 13-acetylbaccatin III by C9 oxidation (Fig. 3 and Fig. 17). The 13-acetylbaccatin III could also be generated from baccatin III by C13 acetylation³⁰. Similarly, supplying *N. benthiamana* expressed TBT, DBAT and TAT with deacetyl-9-dihydro-13-acetylbaccatin III as an exogenously supplied substrate resulted in the production of baccatin III (Fig. 18). A mechanism for spontaneous formation of the oxetane ring has been suggested previous research^{14,27} (Fig. 19). Here, the spontaneous transfer of the deacetyl-1-hydroxybaccatin I oxetane ring to deacetyl- 9-dihydro-13-acetylbaccatin III was also observed (Fig. 20). In the presence of the co-expressed TBT, DBAT, TAT and T9oxidase, deacetyl-1-hydroxybaccatin I could produce baccatin III. Moreover, neither deacetylated taxusin nor taxa-4(20),11-dien-5α-ol, could be used for baccatin III biosynthesis in our feeding experiment same as previous research (Figs. 21-22)^{14,27}.

### Example 5 - Identification of two missing hydroxylases

The activity of the next missing step is predicted to be a taxoid 9α-hydroxylase. A *Ginkgo biloba* P450 was reported to have the function of taxoid 9α-hydroxylase which could catalyze the 9α-hydroxylation of the taxoid sinenxan A³¹. Three P450s with high sequence similarity to GbT9αOH could be found. In addition, based on the transcriptomic analysis, twelve new cytochrome P450s were found with high sequence similarity to the other five taxadiene hydroxylation enzymes including three potential T9αOHs. Thus, all potential proteins were screened to determine if they were capable to act as distinct hydroxylases by expressing the selected enzymes alongside nsTXS1, nsGGPS, 4β-C20 epoxidase, HMGR, and P19 in *N. benthamiana* leaves (Fig. 23). The expression CYP725A22-1 was found to significantly reduce the levels of the 4β,20-epoxide- taxadiene and to produce novel metabolic intermediates in m/z 304 by GC-MS, mass consistent with C5 or C10 hydroxylation (Fig. 23). This peak has distinct mass spectral profiles and retention time with all five characterized hydroxylases. To confirm this taxoid 9α-hydroxylase, it was co-expressed with T9α oxidase to oxidase the hydroxyl-C9 with m/z 302 by GC-MS (Fig. 23).

Given that both T7βOH and T2αOH were found to display activity using taxusin as substrate³², here, twelve new cytochrome P450s were independently expressed in *N. benthamiana* leaves which were subsequently fed with Taxusin. Hydroxylased taxusin, which has a distinct retention time but the same spectral profile as 7 β-taxusin and 2 α-taxusin, were generated by CYP725A23-1 (Fig. 24). To confirm the function of theT9aOH and T1βOH candidates within the paclitaxel pathway, all the identified enzymes of baccatin III biosynthesis (i.e. the 13 known enzymes plus these four novel enzymes), were expressed in *N. benthamiana* leaves as described below.

### Example 6- Engineering baccatin III biosynthesis

The use of heterologous hosts for paclitaxel production represents a research priority, given the complexity and associated costs of the current alternative approach based on semisynthesis. In this regard, *Arabidopsis* and *Nicotiana* are expected to offer a more favorable environment for the functional expression of CYP450s and the other genes involved in the biosynthesis of paclitaxel pathway intermediates. The two main bottlenecks hindering developments in the elucidation of the full taxol pathway are represented by the lack of detection of many putative intermediates and the insufficient data concerning the localization and the transport of these intermediates at the subcellular level. Thus, all the baccatin III biosynthetic genes were engineered including the *P19, HMGR,* and *BZO,* so that their products were located in either the cytosol or the ER membrane. The engineered DBAT which has been demonstrated to possess superior catalytic properties being estimated to be 60 times more efficient³³, was also used for this engineering. Although the co-transformation efficiency of these three large plasmids remained low, it was found that both 10-deacetylbaccatin III and baccatin III were produced in all replications in *N. benthamiana* leaves (Fig. 4a and Figs. 25-26). Although the accumulation of 10-deacetylbaccatin III is relatively low (~15.40 ng/g FW), and the accumulation of baccatin III could reach ~154.84ng/g FW with in *N. benthamiana,* respectively, and in *Taxus* needles is around ~225.32 ng/g FW. Despite the low yield, these results showed that 10-Deacetylbaccatin III and baccatin III are produced in a heterologous system, thereby having defined an entire minimal pathway for its biosynthesis (Fig. 4 and Figs. 25-26). As a final experiment regarding the pathway structure, a so-called dropout experiment was carried out in which the entire pathway of baccatin III biosynthesis was expressed in *N. benthamiana* leaves with the exception of one gene at a time. In no instance was the baccatin III produced, these confirming that each gene is necessary under essential for baccatin III biosynthesis (Fig. 5b).

### Example 7 - Identification of enzymes catalyzing the last steps of paclitaxel biosynthesis

In the final steps of paclitaxel biosynthesis, the most pressing challenges are to elucidate the reactions leading to the side chain attachment of β-phenylalanoyl-coA to baccatin III and its downstream modifications (Fig. 5a). This part of the pathway is known to include the conversion of α-phenylalanine to β-phenylalanine by phenylalanine aminomutase (PAM)³⁴, the activation of β-phenylalanine to a CoA ester by a β-phenylalanine-CoA ligase (PCL), the binding of the side chain by the enzyme BAPT³⁵, the hydroxylation of the side chain at carbon 2' by an as yet uncharacterized 12'αOH¹⁸, and the benzoylation of the product of this reaction by 3DBTNBT³⁶. An in-depth computational and experimental analysis of a previously analyzed cDNA-AFLP dataset¹⁶ was recently conducted, leading to the identification of transcript TB506 (CYP73A171) as a putative T2'αOH¹⁸. Molecular docking was conducted to confirm the binding of such a large substrate as 3'N-dehydroxydebenzoyltaxol with possible docking sites being recognized. Here it was identified that this T2'αOH was sub-cellularly-localized to the ER membrane. Intriguingly, although T2'αOH activity was confirmed in *Pisum sativum* protoplasts, T2'αOHs were not yet employed in metabolic engineering strategies. Here, *Arabidopsis* benzoate-CoA ligase (BZO) and *Taxus* BAPT, DBTNBT and T2'αOH were overexpressed in *N*. *benthamiana* leaves and co-infiltrated with benzoic acid, β-phenylalanine-CoA and baccatin III.

Following these experiments, low levels of paclitaxel accumulated in the *N. benthamiana* leaves (Fig. 5b and Fig. 27). This was confirmed by comparison of mass spectra to those of an authentic standard.

Given that one *P. chrysogenum* enzyme (Pc21g30650) had been characterized as β-phenylalanine-CoA ligase³⁷, this gene was used to replace the *Taxus* homologs which could produce paclitaxel (Fig. 5b). Blasting the protein sequence of Pc21g30650 against the *Taxus* genome, seven acyl-activating enzymes (AAE) were identified, including three AAEs from the transcriptomics dataset of the three *Taxus* species²³ (Table 1). Given that the products of most paclitaxel biosynthesis genes are subcellular-localized at the ER membrane, it seems likely that the active PCL will also be found around the ER membrane (Fig. 28). Similar to TSaOH and taxadiene-5a-ol-O-acetyl transferase (TAT) enzymes, this enzyme was additionally localized to the ER membrane (Fig. 28).

To identify the native Taxus benzoate-CoA ligase (BZO), seven co-expressed AAEs were expressed with the suite of enzymes described above (Fig. 5a), following feeding with benzoic acid, L-phenylalanine and baccatin III, a low level of paclitaxel accumulation was detected in the leaves of *N. benthamiana* only on the overexpression of TAAE16 (Fig. 5b and S Fig. 27). Thus, after the identification of this PCL and of the T2'αOH, and the elucidation of the missing steps in baccatin III biosynthesis, a complete "minimal" pathway for the biosynthesis of paclitaxel can be formulated (Fig. 1). Although yields were too low to successfully reconstitute the entire paclitaxel pathway on one module, the combined analysis as presented herein suggests not only that the pathway is complete, but also that paclitaxel production via synthetic biology approaches is possible.

### Example 8 - Discussion

Despite the occurrence of an enormous chemical diversity (close to 600 reported taxane structures and 21,000 recorded chemicals), only the three taxanes paclitaxel, docetaxel, and cabazitaxel have been developed as drugs for the treatment of various types of cancer³⁸. The bioactivities of most other taxanes have not been well characterized due to their low amount. Identification of the full paclitaxel biosynthesis pathway should allow us to overcome this limitation in enabling us to purify large quantities of the various taxanes for use in pre-clinical and clinical trials. Since the release of sequenced genomes of *Taxus*^{15,19,20}, many taxane P450s and acetyltransferases were suggested to be related to taxane biosynthesis. The application of the strategies presented herein will facilitate future research efforts into the biosynthesis of further useful taxanes.

Although the full pathway was identified, the order of some of the catalytic steps in the pathway remains to be deciphered. Given the very low abundance of the intermediates, the classical approach of ordering the sequence of reactions through isotope tracer experiments does not seem to be feasible. Given that all these enzymes are sub-cellular-localized to the ER membrane, and given that few intermediates can be detected, it is possible that substrate channeling may mediate these reactions^{39,40}. Future investigations are still needed to identify the possibility of enzyme-enzyme assemblies and substrate channeling. Such studies may also provide insight into reaction order.

The metabolic engineering of paclitaxel remains limited to the use of a transient expression system in *N. benthamiana.* Given the low enzyme activities of DBAT, PCL and BAPT, and the toxicity of paclitaxel, both 10-deacetylbaccatin III and baccatin III accumulate to levels 10-20 times greater than paclitaxel in *Taxus*⁴¹*.* Moreover, the expression of a mutant *P.chrysogenum* phenylalanine-CoA ligase could significantly increase catalytic efficiency³⁷. Given that the full pathway is identified herein, future engineering work on the BAPT and PCL will likely greatly improve the efficiency of the paclitaxel biosynthesis in stably transformed plants.

Through the combined use of metabolomic analyses and heterologous expression in *N. benthamiana,* it was possible to rapidly establish the complete overview of the complex biosynthetic pathway of paclitaxel, a plant-derived drug of historical and contemporary importance. By combining biosynthetic genes from the medicinal plant *Taxus* with enzymes co-opted from other pathways, *de novo* biosynthetic production of both 10-deacetylbaccatin III and baccatin III in this commonly used model plant is demonstrate herein. Ultimately, the results herein not only provide a metabolic route to the production of paclitaxel, but also highlight a powerful strategy for accelerating the discovery and engineering of natural product biosynthesis in medicinal plants.

### Example 9- Material and Methods

### Data reporting

No statistical methods were used to predetermine sample size. The experiments were not randomized and the investigators were not blinded to allocation during experiments and outcome assessment.

### Chemicals and reagents

Unless stated otherwise, general chemicals and reagents, including 10-deacetylbaccatin III (Sigma, D3676), baccatin III (Sigma, B8154), paclitaxel (Sigma, T7402), L-phenylalanine (Sigma, P2126), DL-β-phenylalanine (Sigma, 159492), β-phenylalanoyl-CoA (TransMIT, No. P038), taxusin (P) (LGC, CDX-00020082-010), 1-hydroxybaccatin I (TMO-TN2540, TargetMol) and 9-dihydro-13-acetylbaccatin III (TMO-T5132, TargetMol) and benzoic acid (Sigma, 242381) were purchased commercially.

### Plant growth

*N. benthamiana* plants were maintained on growth shelves at the Max Planck Institute of Molecular Plant Physiology with a 16-h light and 8-h dark cycle at 22°C. Plants were grown for 4-5 weeks before *Agrobacterium* infiltration, with periodic watering, as needed. *T. baccata* and *T. cuspidata* plant materials were provided by botanical garden of the University of Potsdam. Needles of *T.baccata* and *T*. cuspidata were immediately snap-frozen in liquid nitrogen after removal from the plant and stored at -80 °C until further use.

### Transcriptomic analysis

Additional candidate biosynthetic genes involved in the biosynthesis of paclitaxel were obtained from four published *Taxus* plants RNA-seq datasets (MeJA induced *Taxus* cell lines^{1,2}, three tissues³, three species⁴ and ten-year-old culture compared with new culture⁵) using the thirteen already characterized paclitaxel biosynthesis genes as baits. The top correlated genes were selected from the different datasets and used for the overlap analysis using heatmap.The similar changed genes are selected from different database and did the overlap analysis. Only candidates were selected the which had the possible function of P450s, by characterized hydroxylases, phenylalanine-CoA ligase, by Pc21g30650, taxane 9α-dioxygenase and C4β-C20epoxidase by comparing the sequence homology of similar functional proteins.

### cDNA cloning and vector construction

cDNA of the *T.baccata* needles was generated according to the previous publication¹. The forty-one paclitaxel biosynthesis genes (Table 1) with *Taxus* P450 reductase (CPR), were cloned from -DNA of *T.baccata* by PCR-based Gateway BP cloning using the pDONR207 Donor vector (Thermo Fisher Scientific, Waltham, MA).

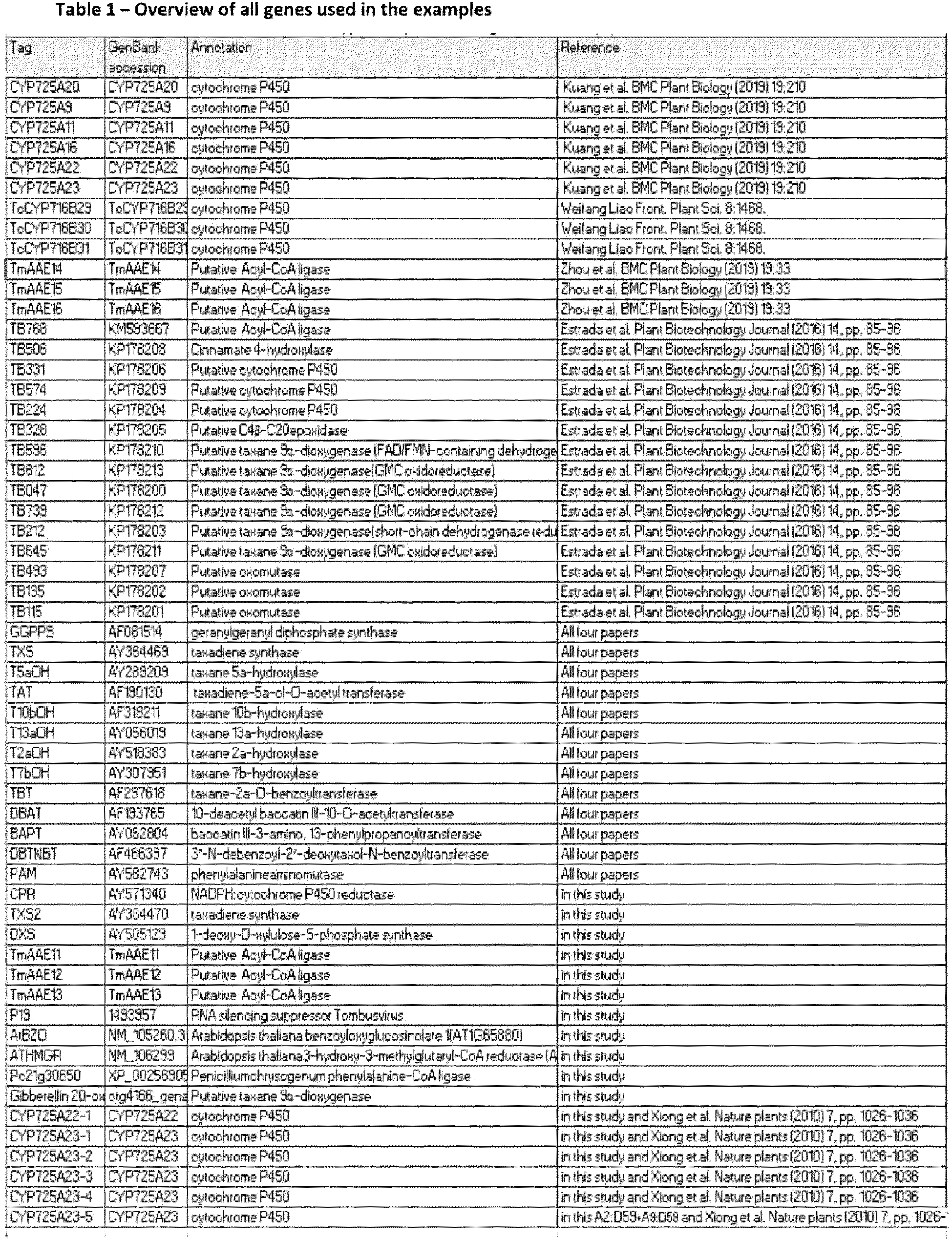

Similarly, ATHMGR and ATBZO were cloned from cDNA of *Arabidopsis.* P19 protein was cloned from pBIN61-P19. Pc21g3065 was cloned from *Penicillium chrysogenum⁶.* The gene-specific primers used did not include a stop codon to ensure C-terminal fusion of tags according to the previous protocol⁷. For subcellular localization, all genes were subcloned into PK7FWG2 with a C-terminal GFP tag. The pBin-ER-mCherry was used as the subcellular localization marker⁸. All the paclitaxel biosynthesis genes were subsequently cloned under the control of the 35S promoter and terminator by gene specific primers with stop codon by In-Fusion (Takara Bio), while both CPR and AtBZO were linked with the CsMV promoter and terminator of Arabidopsis actin 8. Every four genes were linked by two step In-fusion (Takara Bio) using two pairs of promoter and terminator specific primers. Finally, these genes were subcloned into pK7m34GW2-8m21GW3 or pK7WG2 (VIB) using the LR reaction-based construction of expression vectors.

### Agroinfiltration

Frozen stocks of AGL1 *Agrobacterium* Electrocompetent Cells were added to YEB-medium with Carb+ (carbenicillin 25 mg/L) and Rif+ (rifampicin 20 mg/L) and incubated at 28°C overnight. The cells were centrifuged for 30 sec at 20000 g at 4°C and washed with 1ml and 500 µl ice-cold water. The cells were finally resolved in 200 µl of water (these cells are subsequently termed the *Agrobacteria competent cells*)*.* Around 100ng of expression plasmid was added into a 2ml tube with 45 µl of *Agrobacteria* competent cells on ice for 5 mins. The solution was electrically shocked in cuvettes and maintained at 28 °C with 1ml YEB medium. After shaking for 1~2 hours at 28°C at 250rpm, the cells were plated on a YEB plate (Carb+, Rif+ and appropriate antibiotics) and incubated at 28°C for 2 to 3 days.

The *Agrobacterium* were grown on YEB-induced medium plates at 28°C for 24-36h⁹. The cells were scratched and resuspended in 500 µl washing solution (10mM MgCl₂, 100µM acetosyringone). After shortly vortex, the 100 µl resuspended *Agrobacteria* were diluted 10 times to measure the OD₆₀₀ (should be equal to or greater than 12). The *Agrobacteria* were finally diluted to OD₆₀₀ = 1 in infiltration solution (¼MS [pH=6.0], 1% Sucrose , 100µM acetosyringone, 0.005% (v/v, 50µL/L) Silwet L-77). The *Agrobacteria* were infiltrated into *N. benthamiana* leaves using a 1 mL plastic syringe, kept in the light to dry the leaves (1 hour), and then subsequently kept in the dark for 3-4 days at room temperature. Unless stated otherwise, each experimental condition being tested consisted of at least two replicates, with each replicate corresponding to an independently infiltrated leaf. Each leaf among these two replicates was selected from a different *N. benthamiana* plant, such that no replicates for one experimental condition were taken from the same plant. In general, infiltrated leaves were harvested 4-5 days after infiltration, snap-frozen in liquid nitrogen and stored at -80°C for downstream processing.

### Confocal analysis

Constructs were transient expressed in the *N. benthamiana* leaves by agro-infiltration as mentioned above for the protein co-sub localization analysis. Confocal images were taken using a DM6000B/SP8 confocal laser scanning microscope.

### Gas chromatography-mass spectrometry (GC-MS)

For preparing GC-MS analysis of taxa-4(5),11(12)-diene, iso-OCT, OCT and taxadiene-5α-ol, plant materials (needles from *T.baccata*) were collected and powdered with liquid nitrogen. According to previously published protocol¹⁰, around 1g of plant material was extracted three times by 1 ml hexane with 20 mins shaking and 20 minutes sonication cycles. Samples were then extracted once with hexane:ethyl acetate mixture (4:1 v/v). The organic solvent phases were combined and dried under a stream of nitrogen. 1 µl of the sample was injected into GC-MS in splitless mode in Thermo Scientific TRACE 1300 Series Gas Chromatograph by ZB-5 column (phenomenex). The retention time of taxa-4(5),11(12)-diene (m/z 272), OCT (m/z 288) and and taxadiene-5α-ol (m/z 288) was calculated from the positive control of yeast extraction¹¹.

To prepare GC-MS profiling analysis other intermediates from 4β,20-taxadiene and 4β,20-taxadiene-5α-ol, plant material were collected and powdered in liquid nitrogen. Around 1g of plant materials were extracted by 650 µl ethyl acetate with 30 mins sharking and 10 mins sonication cycles. After centrifuging at 16000*g*, 5 mins, the 600 µl supernatant was transferred to 2ml tubers. Subsequently, 500 µl ethyl acetate was added with vertexing and sonication with replication. After repeating this step and collecting all the ethyl acetate extraction (around 1600 µl), the supernatants were dried under a stream of nitrogen gas. Given that a high concentration of chlorophyll results in low-quality GC-MS profiling, the dried extracts were treated with 100% methanol for 10 mins vortexing and 10 mins sonication at 4°C. After adding 200 µl CHCl3, 600 µl ddH₂O was added to extract the polar metabolites. The supernatant was subsequently dried under a stream of gas. Trimethylsilylation was performed by adding 50 µL of a mixture of N,O-bis(trimethylsilyl)trifluoroacetamide (BSTFA) and an n-alkane standard mix in hexane (7:1, v/v) followed by incubation at 37°C for 30 min with gentle shaking (800 rpm). 1 µl of the sample was injected into GC-MS in splitless mode. 1 µl of the sample was injected into GC-MS in splitless mode.

Metabolite profiling was performed as detailed previously by gas chromatography coupled to electron impact ionization/time-of-flight mass spectrometry (GC-EI/TOF-MS) using an Agilent 6890N24 gas chromatograph (Agilent Technologies) connected to a Pegasus III time-of-flight mass spectrometer (LECO Instrumente GmbH, Mönchengladbach, Germany)¹². Data analysis was performed with Xcalibur^{™} software (Thermo Scientific^{™}). The retention times of 4β,20-taxadiene (m/z 288, RT, 21.70), 4β,20-taxadiene-5α-triol (m/z 376, 1TMS RT, 22.33), were determined comparing peaks with the expected masses in the samples and negative controls.

### Ultra-high-performance liquid chromatography high-resolution mass spectrometry (UHPLC-HRMS)

To prepare UHPLC-HRMS profiling analysis, plant material was collected and powdered in liquid nitrogen. Around 50mg of plant material was extraced by methyl-*tert*-butyl-ether (MTBE):methanol (MeOH) extraction method¹³. Both the upper and lower phase were dried on a vacuum concentrator, resuspended in 200 µl acetonitrile:isopropanol (70:30, upper phase) or 50% methanol (lower phase), and transferred to a glass vial for UHPLC-HRMS for metabolomic profiling. The UHPLC-HRMS system consisted of a Waters Acquity UPLC coupled to an Exactive Orbitrap mass detector according to previously published protocol^{14,15}. The UPLC system was equipped with an HSS T3 C₁₈ reversed-phase column (100 × 2.1 mm i.d., 1.8-µm particle size; Waters) that was operated at a temperature of 40°C. The mobile phases consisted of 0.1% formic acid in water (Solvent A) and 0.1% formic acid in acetonitrile (Solvent B). The flow rate of the mobile phase was 400 µl/min, and 2 µL of the sample was injected. The UPLC was connected to an Exactive Orbitrap (Thermo Fisher Scientific) via a heated electrospray source (Thermo Fisher Scientific). The spectra were recorded using the full scan mode of positive ion detection modes, covering a mass range from *m*/*z* 100 to 1500. The resolution was set to 25,000, and the maximum scan time was set to 250 ms. The sheath gas was set to a value of 60, while the auxiliary gas was set to 35. The transfer capillary temperature was set to 150°C, while the heater temperature was adjusted to 300°C. The spray voltage was fixed at 3 kV, with a capillary voltage and a skimmer voltage of 25 and 15 V, respectively. MS spectra were recorded from minute 0 to 19 of the UPLC gradient. Molecular masses, retention time, and associated peak intensities were extracted from the raw files using Xcalibur^{™} software (Thermo Fisher Scientific). Metabolite identification and annotation were performed using standard compounds and the monoisotopic mass of predicted compounds. Data are reported herein in a manner compliant with the literature standards^{16,17}.

### Chemical Reaction Experiments

In a typical mCBPA reaction¹⁸, taxadiene (1 mg, 0.0036 mmol) was weighed into a 14 mL pyrex glass tube containing CH₂Cl₂ (1 mL), NaHCO₃ (0.5 mg, 0.05 mmol). A magnetic stir bar was added and placed in a salt water/ice bath on a magnetic stir plate. The reaction was initiated with the addition of mCPBA (20 µL of a 25 mmolar stock, calculated assuming a nominal purity of 70%) and was allowed to proceed for 2 h. The crude product mixture was washed twice with saturated sodium carbonate and once with NaS₂O₃. The organic layer was then removed and dried to completion and the products subjected to analysis. A typical DMDO reaction¹⁸ was carried out by adding 0.34 mg of taxadiene (0.00125 mmol) to 250 µL of EA and 250 µL of water (buffered with 0.66 mg sodium carbonate) with 10 µL of acetate. Oxone in water (4.8 mg in 1 mL) and a solution of acetone and sodium carbonate in water (18 µL and 0.66 mg, respectively, in 1 mL) were added dropwise as separate solutions over 2 h. Sodium carbonate was chosen as the final buffering reagent as the reactions exhibited maximum yield when operated at high pH. Direct analysis of the organic layer via GCMS yielded identical product ratios, indicating wash steps did not alter product distribution.

The deacetylation of taxusin, 1-hydroxybaccatin I and 9-dihydro-13-acetylbaccatin III was performed by global reductive removal of the acetates with LiAlH₄ as describe by previous research ¹⁹and confirmed by UHPLC-HRMS. Both taxusin-4β,20-epoxide and taxusin-4α,20-epoxide were synthesized from taxusin by peracetic acid as describe by previous research²⁰ and measured by an Acquity UPLC system using an RP C8 column and analyzed by MS¹³.

### Enzyme assay

The epoxidase was transiently expressed in the *N. benthamiana* leaves by agro-infiltration. A leaf disc was feeded with Taxusin in ¼ MS medium for 12h reaction. Samples were extracted by MTBE method and measured by an Acquity UPLC system using an RP C8 column and analyzed by MS¹³.

### References main text and examples 1 to 8

1 Lucas, H. Ueber ein in den Blättern von Taxus baccata L. enthaltenes Alkaloid (das Taxin). Archiv der Pharmazie 135, 145-149 (1856).
2 Wani, M. C., Taylor, H. L., Wall, M. E., Coggon, P. & McPhail, A. T. Plant antitumor agents. VI. Isolation and structure of taxol, a novel antileukemic and antitumor agent from Taxus brevifolia. Journal of the American Chemical Society 93, 2325-2327 (1971).
3 Yang, C.-P. H. & Horwitz, S. B. Taxol®: the first microtubule stabilizing agent. International journal of molecular sciences 18, 1733 (2017).
4 Fischer, J. & Ganellin, C. R. Analogue-based drug discovery. Chemistry International-Newsmagazine for IUPAC 32, 12-15 (2010).
5 Onrubia, M. et al. Coronatine, a more powerful elicitor for inducing taxane biosynthesis in Taxus media cell cultures than methyl jasmonate. Journal of plant physiology 170, 211-219 (2013).
6 Li, J. et al. Chloroplastic metabolic engineering coupled with isoprenoid pool enhancement for committed taxanes biosynthesis in Nicotiana benthamiana. Nature communications 10, 1-12 (2019).
7 Nicolaou, K. et al. Total synthesis of taxol. Nature 367, 630-634 (1994).
8 Mutanda, I., Li, J., Xu, F. & Wang, Y. Recent Advances in Metabolic Engineering, Protein Engineering, and Transcriptome-Guided Insights Toward Synthetic Production of Taxol. Frontiers in Bioengineering and Biotechnology 9 (2021).
9 Nazhand, A. et al. Rewiring cellular metabolism for heterologous biosynthesis of Taxol. Natural product research 34, 110-121 (2020).
10 Roberts, S. C. Production and engineering of terpenoids in plant cell culture. Nature chemical biology 3, 387-395 (2007).
11 Zhou, K., Qiao, K., Edgar, S. & Stephanopoulos, G. Distributing a metabolic pathway among a microbial consortium enhances production of natural products. Nature biotechnology 33, 377-383 (2015).
12 Köksal, M., Jin, Y., Coates, R. M., Croteau, R. & Christianson, D. W. Taxadiene synthase structure and evolution of modular architecture in terpene biosynthesis. Nature 469, 116-120 (2011).
13 De La Peña, R. & Sattely, E. S. Rerouting plant terpene biosynthesis enables momilactone pathway elucidation. Nature chemical biology 17, 205-212 (2021).
14 Guerra-Bubb, J., Croteau, R. & Williams, R. M. The early stages of taxol biosynthesis: an interim report on the synthesis and identification of early pathway metabolites. Natural product reports 29, 683-696 (2012).
15 Zhang, Y., Scossa, F. & Fernie, A. R. The genomes of Taxus species unveil novel candidates in the biosynthesis of taxoids. Molecular Plant 14, 1773-1775 (2021).
16 Ramirez-Estrada, K. et al. Transcript profiling of jasmonate-elicited Taxus cells reveals a β-phenylalanine-CoA ligase. Plant biotechnology journal 14, 85-96 (2016).
17 Onrubia, M. et al. Taximin, a conserved plant-specific peptide is involved in the modulation of plant-specialized metabolism. Plant biotechnology journal 12, 971-983 (2014).
18 Sanchez-Muñoz, R. et al. A novel hydroxylation step in the taxane biosynthetic pathway: a new approach to paclitaxel production by synthetic biology. Frontiers in bioengineering and biotechnology 8, 410 (2020).
19 Cheng, J. et al. Chromosome-level genome of Himalayan yew provides insights into the origin and evolution of the paclitaxel biosynthetic pathway. Molecular Plant 14, 1199-1209 (2021).
20 Xiong, X. et al. The Taxus genome provides insights into paclitaxel biosynthesis. Nature Plants 7, 1026-1036 (2021).
21 Mutwil, M. Computational approaches to unravel the pathways and evolution of specialized metabolism. Current Opinion in Plant Biology 55, 38-46 (2020).
22 Kuang, X., Sun, S., Wei, J., Li, Y. & Sun, C. Iso-Seq analysis of the Taxus cuspidata transcriptome reveals the complexity of Taxol biosynthesis. BMC plant biology 19, 1-16 (2019).
23 Zhou, T. et al. Transcriptome analyses provide insights into the expression pattern and sequence similarity of several taxol biosynthesis-related genes in three Taxus species. BMC plant biology 19, 1-10 (2019).
24 Liao, W. et al. Transcriptome assembly and systematic identification of novel cytochrome P450s in Taxus chinensis. Frontiers in plant science 8, 1468 (2017).
25 Ajikumar, P. K. et al. Isoprenoid pathway optimization for Taxol precursor overproduction in Escherichia coli. Science 330, 70-74 (2010).
26 Edgar, S., Li, F.-S., Qiao, K., Weng, J.-K. & Stephanopoulos, G. Engineering of taxadiene synthase for improved selectivity and yield of a key taxol biosynthetic intermediate. ACS Synthetic Biology 6, 201-205 (2017).
27 Ondari, M. E. & Walker, K. D. The taxol pathway 10-O-acetyltransferase shows regioselective promiscuity with the oxetane hydroxyl of 4-deacetyltaxanes. Journal of the American Chemical Society 130, 17187-17194 (2008).
28 Rontein, D. et al. CYP725A4 from yew catalyzes complex structural rearrangement of taxa-4 (5), 11 (12)-diene into the cyclic ether 5 (12)-oxa-3 (11)-cyclotaxane. Journal of Biological Chemistry 283, 6067-6075 (2008).
29 Kaspera, R., Cape, J. L., Faraldos, J. A., Ketchum, R. E. & Croteau, R. B. Synthesis and in vitro evaluation of taxol oxetane ring D precursors. Tetrahedron letters 51, 2017-2019 (2010).
30 Chau, M., Walker, K., Long, R. & Croteau, R. Regioselectivity of taxoid-O-acetyltransferases: heterologous expression and characterization of a new taxadien-5α-ol-O-acetyltransferase. Archives of biochemistry and biophysics 430, 237-246 (2004).
31 Zhang, N. et al. Molecular cloning and characterization of a cytochrome P450 taxoid 9a-hydroxylase in Ginkgo biloba cells. Biochemical and biophysical research communications 443, 938-943 (2014).
32 Chau, M., Jennewein, S., Walker, K. & Croteau, R. Taxol biosynthesis: molecular cloning and characterization of a cytochrome P450 taxoid 7β-hydroxylase. Chemistry & biology 11, 663-672 (2004).
33 You, L.-F. et al. Enhanced catalytic activities and modified substrate preferences for taxoid 10β-O-acetyl transferase mutants by engineering catalytic histidine residues. Biotechnology letters 40, 1245-1251 (2018).
34 Walker, K. D., Klettke, K., Akiyama, T. & Croteau, R. Cloning, heterologous expression, and characterization of a phenylalanine aminomutase involved in Taxol biosynthesis. Journal of Biological Chemistry 279, 53947-53954 (2004).
35 Walker, K., Fujisaki, S., Long, R. & Croteau, R. Molecular cloning and heterologous expression of the C-13 phenylpropanoid side chain-CoA acyltransferase that functions in Taxol biosynthesis. Proceedings of the National Academy of Sciences 99, 12715-12720 (2002).
36 Walker, K., Long, R. & Croteau, R. The final acylation step in taxol biosynthesis: cloning of the taxoid C13-side-chain N-benzoyltransferase from Taxus. Proceedings of the National Academy of Sciences 99, 9166-9171 (2002).
37 Koetsier, M. J., Jekel, P. A., Wijma, H. J., Bovenberg, R. A. & Janssen, D. B. Aminoacylcoenzyme A synthesis catalyzed by a CoA ligase from Penicillium chrysogenum. FEBS letters 585, 893-898 (2011).
38 Lange, B. M. & Conner, C. F. Taxanes and taxoids of the genus Taxus-A comprehensive inventory of chemical diversity. Phytochemistry 190, 112829 (2021).
39 Zhang, Y. & Fernie, A. R. Metabolons, enzyme-enzyme assemblies that mediate substrate channeling, and their roles in plant metabolism. Plant Communications 2, 100081 (2021).
40 Pareek, V., Sha, Z., He, J., Wingreen, N. S. & Benkovic, S. J. Metabolic channeling: predictions, deductions, and evidence. Molecular cell 81, 3775-3785 (2021).
41 McElroy, C. & Jennewein, S. in Biotechnology of natural products 145-185 (Springer, 2018).
42 Walls, L. E., Martinez, J. L. & Rios-Solis, L. Enhancing Saccharomyces cerevisiae taxane biosynthesis and overcoming nutritional stress-induced pseudohyphal growth. Microorganisms 10, 163 (2022).
43 Koepp, A. E. et al. Cyclization of geranylgeranyl diphosphate to taxa-4 (5), 11 (12)-diene is the committed step of taxol biosynthesis in Pacific yew. Journal of Biological Chemistry 270, 8686-8690 (1995).
44 Jennewein, S., Rithner, C. D., Williams, R. M. & Croteau, R. B. Taxol biosynthesis: taxane 13α-hydroxylase is a cytochrome P450-dependent monooxygenase. Proceedings of the National Academy of Sciences 98, 13595-13600 (2001).
45 Chau, M. & Croteau, R. Molecular cloning and characterization of a cytochrome P450 taxoid 2α-hydroxylase involved in Taxol biosynthesis. Archives of Biochemistry and Biophysics 427, 48-57 (2004).

### References example 9

1 Ramirez-Estrada, K. et al. Transcript profiling of jasmonate-elicited Taxus cells reveals a β-phenylalanine-CoA ligase. Plant biotechnology journal 14, 85-96 (2016).
2 Xiong, X. et al. The Taxus genome provides insights into paclitaxel biosynthesis. Nature Plants 7, 1026-1036 (2021).
3 Kuang, X., Sun, S., Wei, J., Li, Y. & Sun, C. Iso-Seq analysis of the Taxus cuspidata transcriptome reveals the complexity of Taxol biosynthesis. BMC plant biology 19, 1-16 (2019).
4 Zhou, T. et al. Transcriptome analyses provide insights into the expression pattern and sequence similarity of several taxol biosynthesis-related genes in three Taxus species. BMC plant biology 19, 1-10 (2019).
5 Liao, W. et al. Transcriptome assembly and systematic identification of novel cytochrome P450s in Taxus chinensis. Frontiers in plant science 8, 1468 (2017).
6 Koetsier, M. J., Jekel, P. A., Wijma, H. J., Bovenberg, R. A. & Janssen, D. B. Aminoacylcoenzyme A synthesis catalyzed by a CoA ligase from Penicillium chrysogenum. FEBS letters 585, 893-898 (2011).
7 Zhang, Y. et al. Rapid identification of protein-protein interactions in plants. Current protocols in plant biology 4, e20099 (2019).
8 Nelson, B. K., Cai, X. & Nebenführ, A. A multicolored set of in vivo organelle markers for colocalization studies in Arabidopsis and other plants. The Plant Journal 51, 1126-1136, doi:https://doi.org/10.1111/j.1365-313X.2007.03212.x (2007).
9 Zhang, Y. et al. A highly efficient agrobacterium-mediated method for transient gene expression and functional studies in multiple plant species. Plant communications 1, 100028 (2020).
10 Li, J. et al. Chloroplastic metabolic engineering coupled with isoprenoid pool enhancement for committed taxanes biosynthesis in Nicotiana benthamiana. Nature communications 10, 1-12 (2019).
11 Walls, L. E., Martinez, J. L. & Rios-Solis, L. Enhancing Saccharomyces cerevisiae taxane biosynthesis and overcoming nutritional stress-induced pseudohyphal growth. Microorganisms 10, 163 (2022).
12 Erban, A., Schauer, N., Fernie, A. R. & Kopka, J. in Metabolomics 19-38 (Springer, 2007).
13 Salem, M. A. et al. An improved extraction method enables the comprehensive analysis of lipids, proteins, metabolites and phytohormones from a single sample of leaf tissue under water-deficit stress. The Plant Journal 103, 1614-1632 (2020).
14 Perez de Souza, L., Alseekh, S., Naake, T. & Fernie, A. Mass spectrometry-based untargeted plant metabolomics. Current protocols in plant biology 4, e20100 (2019).
15 Alseekh, S. et al. Identification and mode of inheritance of quantitative trait loci for secondary metabolite abundance in tomato. The Plant Cell 27, 485-512 (2015).
16 Alseekh, S. et al. Mass spectrometry-based metabolomics: A guide for annotation, quantification and best reporting practices. Nature methods 18, 747-756 (2021).
17 Perez de Souza, L., Alseekh, S., Scossa, F. & Fernie, A. R. Ultra-high-performance liquid chromatography high-resolution mass spectrometry variants for metabolomics research. Nature Methods 18, 733-746 (2021).
18 Edgar, S. et al. Mechanistic insights into taxadiene epoxidation by taxadiene-5α-hydroxylase. ACS chemical biology 11, 460-469 (2016).
19 Li, H., Horiguchi, T., Croteau, R. & Williams, R. M. Studies on Taxol biosynthesis: preparation of taxadiene-diol and triol derivatives by deoxygenation of taxusin. Tetrahedron 64, 6561-6567 (2008).
20 Kaspera, R., Cape, J. L., Faraldos, J. A., Ketchum, R. E. & Croteau, R. B. Synthesis and in vitro evaluation of taxol oxetane ring D precursors. Tetrahedron letters 51, 2017-2019 (2010).

## Claims

1. A method for producing 4β,20-taxadiene comprising
(a) enzymatically converting taxa-4(5),11(12)-diene with a C4β,C20-epoxidase selected from
(i) a polypeptide comprising or consisting of the amino acid sequence of SEQ ID NO: 3;
(ii) a polypeptide being encoded by a nucleic acid molecule comprising or consisting of the nucleotide sequence of SEQ ID NO: 4;
(iii) a polypeptide having an amino acid sequence being at least 80% identical, preferably at least 90% and most preferably at least 95% identical to the amino acid sequence of SEQ ID NO: 3;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 4; and
(v) a fragment of the polypeptide of any of (i) to (iv) comprising at least 200 amino acids
thereby producing 4β,20-taxadiene.

2. The method of claim 1, further comprising prior to step (a)
(a') enzymatically converting geranylgeranyl diphosphate (GGPP) with a taxadien synthase (EC 4.2.3.17) thereby producing texa-4(5),11(12)-diene, preferably wherein the taxadien synthase is selected from
(i) a polypeptide comprising or consisting of the amino acid sequence of SEQ ID NO: 1;
(ii) a polypeptide being encoded by a nucleic acid molecule comprising or consisting of the nucleotide sequence of SEQ ID NO: 2;
(iii) a polypeptide having an amino acid sequence being at least 80% identical, preferably at least 90% and most preferably at least 95% identical to the amino acid sequence of SEQ ID NO: 1;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 2; and
(v) a fragment of the polypeptide of any of (i) to (iv) comprising at least 600 amino acids.

3. A method for producing 10-deacetylbaccatin III comprising
(a) enzymatically converting 9-di-10-deacetylbaccatin III with an T9α-oxidase selected from
(i) a polypeptide comprising or consisting of the amino acid sequence of SEQ ID NO: 25, 39, 41 or 43;
(ii) a polypeptide being encoded by a nucleic acid molecule comprising or consisting of the nucleotide sequence of SEQ ID NO: 26, 40, 42 or 44;
(iii) a polypeptide having an amino acid sequence being at least 80% identical, preferably at least 90% and most preferably at least 95% identical to the amino acid sequence of SEQ ID NO: 25, 39, 41 or 43;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 26, 40, 42 or 44; and
(v) a fragment of the polypeptide of any of (i) to (iv) comprising at least 300 amino acids
thereby producing 10-deacetylbaccatin III.

4. A method for producing baccatin III comprising
(a) producing 4β,20-taxadiene according to the method of claim 1 or 2;
(b) enzymatically converting 4β,20-taxadiene with a TSaOH hydroxylase (EC 1.14.14.176) thereby producing 4β,20-taxadiene-5α-ol;
(c) enzymatically converting 4β,20-taxadiene-5α-ol with a T2αOH hydroxylase, a T7βOH hydroxylase (EC 1.14.13.147), a T10βOH hydroxylase (EC 1.14.13.76) and a T13αOH hydroxylase (EC 1.14.13.77) thereby producing 4β,20-taxadiene-2α,5α,7β,10β,13α-ol;
(d) enzymatically converting 4β,20-taxadiene-2α,5α,7β,10β,13α-ol with a T9αOH hydroxylase thereby producing 4β,20-taxadiene-2α,5α,7β,9α,10β,13α-ol;
(e) enzymatically converting 4β,20-taxadiene-2α,5α,7β,9α,10β,13α-ol with a T1βOH hydroxylase thereby producing 4β,20-taxadiene-1β,2α,5α,7β, 9α,10β,13α-ol;
(f) enzymatically converting 4β,20-taxadiene-1β,2α,5α,7β, 9α,10β,13α-ol with taxadien-5α-ol-O-acetyl-transferase (TAT, EC 2.3.1.162) thereby producing 4-acetyloxy-4β,20-taxadiene-1β,2α,5α,7β,9α,10β,13α-ol;
(g) enzymatically converting 4-acetyloxy-4β,20-taxadiene-1β,2α,5α,7β,9α,10β,13α-ol with taxane-2α-O-benzoyltransferase (TBT, EC 2.3.1.166) thereby producing 9-di-10-deacetylbaccatin III;
(h) enzymatically converting 9-di-10-deacetylbaccatin III with T9α-oxidase thereby producing 10-deacetylbaccatin III, preferably according to the method of claim 3; and
(i) enzymatically converting 10-deacetylbaccatin III with 10-deacetylbaccatin III-10-O-acatyltransferase (DBAT, EC 2.3.1.167) thereby producing baccatin III.

5. The method of claim 4, wherein one or more of (b') to (i') apply:
(b') the TSaOH hydroxylase is selected from
(i) a polypeptide comprising or consisting of the amino acid sequence of SEQ ID NO: 5;
(ii) a polypeptide being encoded by a nucleic acid molecule comprising or consisting of the nucleotide sequence of SEQ ID NO: 6;
(iii) a polypeptide having an amino acid sequence being at least 80% identical, preferably at least 90% and most preferably at least 95% identical to the amino acid sequence of SEQ ID NO: 5;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 6; and
(v) a fragment of the polypeptide of any of (i) to (iv) comprising at least 300 amino acids;
(c') the T2αOH hydroxylase is selected from
(i) a polypeptide comprising or consisting of the amino acid sequence of SEQ ID NO: 7;
(ii) a polypeptide being encoded by a nucleic acid molecule comprising or consisting of the nucleotide sequence of SEQ ID NO: 8;
(iii) a polypeptide having an amino acid sequence being at least 80% identical, preferably at least 90% and most preferably at least 95% identical to the amino acid sequence of SEQ ID NO: 7;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 8; and
(v) a fragment of the polypeptide of any of (i) to (iv) comprising at least 300 amino acids; and/or
the T7βOH hydroxylase is selected from
(i) a polypeptide comprising or consisting of the amino acid sequence of SEQ ID NO: 9;
(ii) a polypeptide being encoded by a nucleic acid molecule comprising or consisting of the nucleotide sequence of SEQ ID NO: 10;
(iii) a polypeptide having an amino acid sequence being at least 80% identical, preferably at least 90% and most preferably at least 95% identical to the amino acid sequence of SEQ ID NO: 9;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 10; and
(v) a fragment of the polypeptide of any of (i) to (iv) comprising at least 300 amino acids; and/or
the T10βOH hydroxylase is selected from
(i) a polypeptide comprising or consisting of the amino acid sequence of SEQ ID NO: 11;
(ii) a polypeptide being encoded by a nucleic acid molecule comprising or consisting of the nucleotide sequence of SEQ ID NO: 12;
(iii) a polypeptide having an amino acid sequence being at least 80% identical, preferably at least 90% and most preferably at least 95% identical to the amino acid sequence of SEQ ID NO: 11;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 12; and
(v) a fragment of the polypeptide of any of (i) to (iv) comprising at least 300 amino acids; and/or
the T13αOH hydroxylase is selected from
(i) a polypeptide comprising or consisting of the amino acid sequence of SEQ ID NO: 13;
(ii) a polypeptide being encoded by a nucleic acid molecule comprising or consisting of the nucleotide sequence of SEQ ID NO: 14;
(iii) a polypeptide having an amino acid sequence being at least 80% identical, preferably at least 90% and most preferably at least 95% identical to the amino acid sequence of SEQ ID NO: 13;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 14; and
(v) a fragment of the polypeptide of any of (i) to (iv) comprising at least 300 amino acids;
(d') the T9αOH hydroxylase is selected from
(i) a polypeptide comprising or consisting of the amino acid sequence of SEQ ID NO: 15;
(ii) a polypeptide being encoded by a nucleic acid molecule comprising or consisting of the nucleotide sequence of SEQ ID NO: 16;
(iii) a polypeptide having an amino acid sequence being at least 80% identical, preferably at least 90% and most preferably at least 95% identical to the amino acid sequence of SEQ ID NO: 15;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 16; and
(v) a fragment of the polypeptide of any of (i) to (iv) comprising at least 300 amino acids;
(e') the T1βOH hydroxylase is selected from
(i) a polypeptide comprising or consisting of the amino acid sequence of SEQ ID NO: 17;
(ii) a polypeptide being encoded by a nucleic acid molecule comprising or consisting of the nucleotide sequence of SEQ ID NO: 18;
(iii) a polypeptide having an amino acid sequence being at least 80% identical, preferably at least 90% and most preferably at least 95% identical to the amino acid sequence of SEQ ID NO: 17;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 18; and
(v) a fragment of the polypeptide of any of (i) to (iv) comprising at least 300 amino acids;
(f') the TAT is selected from
(i) a polypeptide comprising or consisting of the amino acid sequence of SEQ ID NO: 19;
(ii) a polypeptide being encoded by a nucleic acid molecule comprising or consisting of the nucleotide sequence of SEQ ID NO: 20;
(iii) a polypeptide having an amino acid sequence being at least 80% identical, preferably at least 90% and most preferably at least 95% identical to the amino acid sequence of SEQ ID NO: 19;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 20; and
(v) a fragment of the polypeptide of any of (i) to (iv) comprising at least 300 amino acids;
(g') the TBT is selected from
(i) a polypeptide comprising or consisting of the amino acid sequence of SEQ ID NO: 21;
(ii) a polypeptide being encoded by a nucleic acid molecule comprising or consisting of the nucleotide sequence of SEQ ID NO: 22;
(iii) a polypeptide having an amino acid sequence being at least 80% identical, preferably at least 90% and most preferably at least 95% identical to the amino acid sequence of SEQ ID NO: 21;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 22; and
(v) a fragment of the polypeptide of any of (i) to (iv) comprising at least 300 amino acids;
(h') the T9α-oxidase is selected from
(i) a polypeptide comprising or consisting of the amino acid sequence of SEQ ID NO: 25, 39, 41 or 43;
(ii) a polypeptide being encoded by a nucleic acid molecule comprising or consisting of the nucleotide sequence of SEQ ID NO: 26, 40, 42 or 44;
(iii) a polypeptide having an amino acid sequence being at least 80% identical, preferably at least 90% and most preferably at least 95% identical to the amino acid sequence of SEQ ID NO: 25, 39, 41 or 43;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 26, 40, 42 or 44; and
(v) a fragment of the polypeptide of any of (i) to (iv) comprising at least 300 amino acids; and
(i') the DBAT is selected from
(i) a polypeptide comprising or consisting of the amino acid sequence of SEQ ID NO: 27;
(ii) a polypeptide being encoded by a nucleic acid molecule comprising or consisting of the nucleotide sequence of SEQ ID NO: 28;
(iii) a polypeptide having an amino acid sequence being at least 80% identical, preferably at least 90% and most preferably at least 95% identical to the amino acid sequence of SEQ ID NO: 27;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 28; and
(v) a fragment of the polypeptide of any of (i) to (iv) comprising at least 300 amino acids.

6. A method for producing β-phenylalanoyl-coA (i) from β-phenylalanine by β-phenylalanine coenzyme A ligase (PLC, EC 6.2.1.30), or (ii) from L-phenylalanine by phenylalanine aminomutase (PAM, EC 5.4.3.11) and β-phenylalanine coenzyme A ligase (EC 6.2.1.30),
wherein the β-phenylalanine coenzyme A ligase is selected from
(i) a polypeptide comprising or consisting of the amino acid sequence of SEQ ID NO: 33;
(ii) a polypeptide being encoded by a nucleic acid molecule comprising or consisting of the nucleotide sequence of SEQ ID NO: 34;
(iii) a polypeptide having an amino acid sequence being at least 80% identical, preferably at least 90% and most preferably at least 95% identical to the amino acid sequence of SEQ ID NO: 33;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 34; and
(v) a fragment of the polypeptide of any of (i) to (iv) comprising at least 300 amino acids; and preferably wherein the phenylalanine aminomutase is selected from
(i) a polypeptide comprising or consisting of the amino acid sequence of SEQ ID NO: 31;
(ii) a polypeptide being encoded by a nucleic acid molecule comprising or consisting of the nucleotide sequence of SEQ ID NO: 32;
(iii) a polypeptide having an amino acid sequence being at least 80% identical, preferably at least 90% and most preferably at least 95% identical to the amino acid sequence of SEQ ID NO: 31;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 32; and
(v) a fragment of the polypeptide of any of (i) to (iv) comprising at least 500 amino acids.

7. A method for producing 3'-N-debenzoyl-2'-deoxytaxol comprising
comprising
(a) producing baccatin III according to the method of claims 4 or 5 and/or producing β-phenylalanoyl-coA according to the method of claim 6; and
(b) enzymatically converting baccatin III and β-phenylalanoyl-coA with a baccatin III 13-0-(3-amino-3-phenylpropanoyl) transferase (BAPT, EC 2.3.1.B16), thereby producing 3'-N-debenzoyl-2'deoxytaxol, preferably wherein the BAPT is selected from
(i) a polypeptide comprising or consisting of the amino acid sequence of SEQ ID NO: 29;
(ii) a polypeptide being encoded by a nucleic acid molecule comprising or consisting of the nucleotide sequence of SEQ ID NO: 30;
(iii) a polypeptide having an amino acid sequence being at least 80% identical, preferably at least 90% and most preferably at least 95% identical to the amino acid sequence of SEQ ID NO: 29;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 30; and
(v) a fragment of the polypeptide of any of (i) to (iv) comprising at least 300 amino acids.

8. A method for producing taxol comprising
(a) producing 3'-N-debenzoyl-2'-deoxytaxol according to the method of claim 7;
(b) enzymatically converting 3'-N-debenzoyl-2'-deoxytaxol with a T2'αOH hydroxylase thereby producing 3'-N-debenzoyltaxol, preferably wherein the T2'αOH hydroxylase is selected from
(i) a polypeptide comprising or consisting of the amino acid sequence of SEQ ID NO: 35;
(ii) a polypeptide being encoded by a nucleic acid molecule comprising or consisting of the nucleotide sequence of SEQ ID NO: 36;
(iii) a polypeptide having an amino acid sequence being at least 80% identical, preferably at least 90% and most preferably at least 95% identical to the amino acid sequence of SEQ ID NO: 35;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 36; and
(v) a fragment of the polypeptide of any of (i) to (iv) comprising at least 300 amino acids; and
(c) enzymatically converting 3'-N-debenzoyltaxol with 3'-N-debenzoyltaxol-2'-deoxytaxol-N-benzoyltransferase (DBTNBT) thereby producing taxol, preferably wherein the DBTNBT is selected from
(i) a polypeptide comprising or consisting of the amino acid sequence of SEQ ID NO: 37;
(ii) a polypeptide being encoded by a nucleic acid molecule comprising or consisting of the nucleotide sequence of SEQ ID NO: 38;
(iii) a polypeptide having an amino acid sequence being at least 80% identical, preferably at least 90% and most preferably at least 95% identical to the amino acid sequence of SEQ ID NO: 37;
(iv) a polypeptide being encoded by a nucleic acid molecule comprising or consisting of a nucleotide sequence which is at least 80% identical, preferably at least 90% and most preferably at least 95% identical to the nucleotide sequence of SEQ ID NO: 38; and
(v) a fragment of the polypeptide of any of (i) to (iv) comprising at least 300 amino acids.

9. Use of a C4β,C20-epoxidase as defined in claim 1 for producing 4β,20-taxadiene from texa-4(5),11(12)-diene.

10. Use of an T9α-oxidase as defined in claim 3 for producing 10-deacetylbaccatin III from 9-di-10-deacetylbaccatin Ill.

11. Use of a β-phenylalanine coenzyme A ligase (PLC, EC 6.2.1.30) as defined in claim 6 for producing β-phenylalanoyl-coA from β-phenylalanine.

12. A vector comprising
(I) a nucleic acid molecule encoding a C4β,C20-epoxidase, which nucleic acid molecule is
(a) a nucleic acid molecule encoding a polypeptide comprising or consisting of the amino acid sequence of SEQ ID NO: 3;
(b) a nucleic acid molecule comprising or consisting of the nucleotide sequence of SEQ ID NO: 4;
(c) a nucleic acid molecule comprising or consisting of a nucleic acid molecule encoding a polypeptide having the activity of a catalase the amino acid sequence of which is at least 80% identical, preferably at least 90% and most preferably at least 95% identical to the amino acid sequence of SEQ ID NO: 3;
(d) a nucleic acid molecule encoding a polypeptide comprising or consisting of a nucleotide sequence which is at least 80% identical, preferably at least 90% and most preferably at least 95% identical to the nucleotide sequence of SEQ ID NO: 4;
(e) a fragment of the nucleic acid molecule of any of (i) to (iv) comprising at least 600 nucleic acids; or
(f) the nucleic acid sequence of any of (a) to (e) wherein T is U;
and/or
(II) a nucleic acid molecule encoding a T9α-oxidase, which nucleic acid molecule is
(a) a nucleic acid molecule encoding a polypeptide comprising or consisting of the amino acid sequence of SEQ ID NO: 25, 39, 41 or 43;
(b) a nucleic acid molecule comprising or consisting of the nucleotide sequence of SEQ ID NO: 26, 40, 42 or 44;
(c) a nucleic acid molecule comprising or consisting of a nucleic acid molecule encoding a polypeptide having the activity of a catalase the amino acid sequence of which is at least 80% identical, preferably at least 90% and most preferably at least 95% identical to the amino acid sequence of SEQ ID NO: 25, 39, 41 or 43;
(d) a nucleic acid molecule encoding a polypeptide comprising or consisting of a nucleotide sequence which is at least 80% identical, preferably at least 90% and most preferably at least 95% identical to the nucleotide sequence of SEQ ID NO: 26, 40, 42 or 44;
(e) a fragment of the nucleic acid molecule of any of (i) to (iv) comprising at least 900 nucleic acids, or
(f) the nucleic acid sequence of any of (a) to (e) wherein T is U;
and/or
(III) a nucleic acid molecule encoding a β-phenylalanine coenzyme A ligase (PLC, EC 6.2.1.30), which nucleic acid molecule is
(a) a nucleic acid molecule encoding a polypeptide comprising or consisting of the amino acid sequence of SEQ ID NO: 33;
(b) a nucleic acid molecule comprising or consisting of the nucleotide sequence of SEQ ID NO: 34;
(c) a nucleic acid molecule comprising or consisting of a nucleic acid molecule encoding a polypeptide having the activity of a catalase the amino acid sequence of which is at least 80% identical, preferably at least 90% and most preferably at least 95% identical to the amino acid sequence of SEQ ID NO: 33;
(d) a nucleic acid molecule encoding a polypeptide comprising or consisting of a nucleotide sequence which is at least 80% identical, preferably at least 90% and most preferably at least 95% identical to the nucleotide sequence of SEQ ID NO:34;
(e) a fragment of the nucleic acid molecule of any of (i) to (iv) comprising at least 900 nucleic acids, or
(f) the nucleic acid sequence of any of (a) to (e) wherein T is U.

13. The vector of claim 12 further comprising one or more nucleic acid molecules encoding one or more of the additional enzymes as defined in one or more of claims 2 and 4 to 8.

14. A recombinant organism, a tissue, a cell, or an organelle
(i) recombinantly expressing the C4β,C20-epoxidase as defined in claim 1 and/or the T9α-oxidase as defined in claim 3 and/or the β-phenylalanine coenzyme A ligase (PLC, EC 6.2.1.30) as defined in claim 6 and optionally expressing one or more of the additional enzymes as defined in one or more of claims 2 and 4 to 8,
(ii) comprising the nucleic acid molecule encoding a C4β,C20-epoxidase as defined in claim 13 and/or the nucleic acid molecule encoding an T9α-oxidase as defined in claim 13 and/or the nucleic acid molecule encoding an β-phenylalanine coenzyme A ligase (PLC, EC 6.2.1.30) as defined in claim 13, and optionally comprising one or more nucleic acid molecules encoding one or more of the additional enzymes as defined in one or more of claims 2 and 4 to 8, or
(iii) carrying the vector of claim 12 or 13.

15. The recombinant organism, tissue, cell, or organelle of claim 14, wherein said organism is a plant, fungus, or a bacterium.
